# EUROPÄISCHE PATENTSCHRIFT

(11) **EP 0 285 562 B1**
(45) Veröffentlichungstag und Bekanntmachung des Hinweises auf die Patenterteilung: **28.04.1993**
(21) Anmeldenummer: 88810187.0
(22) Anmeldetag: 22.03.1988
(51) Int. Cl.: C08F 8/00, C08F 8/30, C12N 1/08

(54) **Kunstharz**
Synthetic resin
Résine synthétique

(30) Priorität: 30.03.1987 CH 1214/87
(43) Veröffentlichungstag der Anmeldung: 05.10.1988
(73) Patentinhaber: CIBA-GEIGY AG, 4002 Basel (CH)
(72) Erfinder: Rink, Hans, CH-4125 Riehen (CH)

(56) Entgegenhaltungen:
- DD-A- 141 977
- US-A- 4 623 484

## Beschreibung

Gegenstand der Erfindung ist ein neuartiges Kunstharz zur Anwendung als Träger für die Synthese von Peptiden und Peptidamiden in fester Phase nach dem bekannten allgemeinen Prinzip der Merrifield-Synthese. Das erfindungsgemässe Kunstharz besteht aus dem Grundgerüst eines für eine solche Merrifield-Synthese verwendbaren Polystyrols, welches gegebenenfalls durch Copolymerisation mit 0-5 Mol%, vorzugsweise 1-2 Mol% Divinylbenzol, quervernetzt ist und welches dadurch gekennzeichnet ist, dass es an Benzolringen seines Grundgerüsts durch die Gruppen der Formel 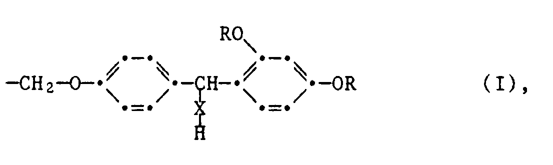
worin X für -O- oder -NH- und R für C₁₋₄-Alkyl steht, substituiert ist. Als X ist im erfindungsgemässen Harz -O- bevorzugt, als Symbol R ist vorzugsweise ein lineares C₁₋₄-Alkyl, in erster Linie Methyl, zu nennen.

Gegenstand der Erfindung ist auch ein Verfahren zur Herstellung des oben charakterisierten Harzes und seine Verwendung zur Herstellung von Peptiden und Peptidamiden, insbesondere solchen, die an der N-terminalen Aminogruppe und/oder an den übrigen funktionellen Gruppen geschützt sind. Gegenstand der Erfindung sind auch entsprechende Harze, in denen der Rest -XH mit der Bedeutung -NH₂ in geschützter Form vorliegt, ferner Harze mit verankerten, gegebenenfalls geschützten Aminosäure-, Peptid- und Peptidamid-Resten, sowie die durch das obengenannte Verfahren hergestellten freien Peptide und Peptidamide, insbesondere die in den Beispielen beschriebenen.

Das Prinzip der im Jahre 1962 veröffentlichten Merrifield-Synthese von peptidischen Verbindungen ist allgemein bekannt. Dabei wird an einem geeignet funktionalisierten Grundpolymer (in der meist verbreiteten Form an einem chloromethylierten Polystyrol) eine an einem Terminus (meistens N-Terminus) geschützte Aminosäure gebunden (verankert). Diese Bindung soll einerseits fest genug sein, um unter verschiedenen Reaktionsbedingungen des synthetischen Aufbaus des Peptids (insbesondere der Abspaltung der N-terminalen Aminoschutzgruppe) unversehrt zu bleiben, andererseits aber die Möglichkeit der Ablösung des fertigen Peptids vom Polymer-Träger unter Bedingungen, die das Produkt nicht beeinträchtigen, gewähren. Die eigentliche Synthese erfolgt dann an dieser am Träger verankerten Aminosäure; dabei wird üblicherweise die N-terminale Schutzgruppe abgespalten, die freigesetzte Aminogruppe durch ein geeignetes, am N-Terminus geschütztes Aminosäurederivat acyliert (d.h. eine neue peptidische Bindung gebildet), und diese Abspaltung der terminalen Schutzgruppe und Acylieren mit einem weiteren Aminosäurerest so oft mal wiederholt, bis eine Aminosäure-Sequenz der gewünschten Länge erreicht ist. Diese Sequenz wird dann durch ein geeignetes Reagens als das fertige Peptid vom Träger abgelöst (abgespalten). Dieser ideale Verlauf ist bei der praktischen Durchführung mit mehreren Nachteilen und Fehlerquellen behaftet, und die Peptidliteratur der letzten 25 Jahre beschäftigt sich fleissig mit diesen Problemen. Eines der schwerwiegendsten liegt darin, dass die aufgebaute Sequenz am Träger nicht von Nebenprodukten befreit werden kann und dass bei jeder weiteren Aufbaustufe eine bereits gebildete Fehlerstruktur (z.B. ein Nebenprodukt mit verkürzter Sequenz) mitreagiert und in die weiteren Stufen mitgeschleppt wird, so dass am Ende das gewünschte Produkt oft nur in einer geringen Menge neben einer überwiegenden Masse von Nebenprodukten ähnlicher Struktur erhalten wird und von diesen aufwendig getrennt werden muss. Diese Situation ist besonders schwierig, wenn die angewendeten Aminosäuren in ihren Seitenketten weitere funktionelle Gruppen enthalten, welche praktisch immer (infolge der verhältnismässig energischen Bedingungen in der Acylierungsoperation) geschützt werden müssen. Diese Schutzgruppen wiederum müssen beständig genug sein, um bei der Freisetzung der N-terminalen Aminogruppe unversehrt zu bleiben. Ein noch weiteres Problem stellen Peptidamide dar, da ihre Bindung zum Träger (im Gegensatz zur Esterbindung gewöhnlicher Peptide) ebenso eine peptidische Bindung ist wie die Bindung zwischen Aminosäuren der ganzen Aminosäuresequenz. Schwierig ist nun die Aufgabe, ein Peptidamid selektiv vom Träger abzulösen unter Erhalt der übrigen peptidischen Bindungen. Aus diesen (und noch weiteren) Gründen wird zurzeit die Festphase-Synthese (Merrifield-Synthese) als eine Methode mit gewissen Vorteilen lediglich beim Aufbau von Peptiden mit höchstens 30 Aminosäuren betrachtet.

Um ihr Anwendungsgebiet zu erweitern, wurde vorgeschlagen, siehe z.B. E. Atherton et al., Proceedings of the 7th American Peptide Symposium, Seiten 163-195, Pierce Chemical Company, Rockford, IL, U.S.A.(1981), ganze Aminosäureteilsequenzen mit geschützten funktionellen Gruppen (einschliesslich der N-terminalen Aminogruppe) als Bausteine an einem Träger zu verankern und mit weiteren geschützten Peptidfragmenten als Bausteinen zu verknüpfen. Nun sollte es möglich sein, auch diese notwendigen Bausteine (Peptidfragmente) durch die Festphase-Synthese am Träger herzustellen. Dies stellt aber eine ganz neue Aufgabe dar. Benötigt wird ein Harz, welches es erlaubt, das synthetisierte Peptidfragment, das aus mehreren geschützten Aminosäureresten besteht, von der Verankerung am Harz unter so milden und/oder selektiven Bedingungen abzulösen, dass weder die N-terminale Schutzgruppe noch die Schutzgruppen sonstiger funktioneller Gruppen (welche notwendigerweise von der ersteren verschiedenartig sein müssen) beeinträchtigt wird. Eine eingehende Diskussion von verschiedenen Vorschlägen und Teillösungen ist in der obgenannten Publikation von Atherton et al. zu finden; diese Autoren selber haben als ihre eigene beste Lösung die Anwendung eines Polyamid-Grundharzes, welches über Norvalin mit 2- oder 3-Methoxy-4-hydroxymethyl-phenoxyessigsäure verbunden ist, vorgeschlagen. Der C-Terminus der ersten (geschützten) Aminosäure ist dann an der Hydroxymethylgruppe durch eine Esterbindung verankert; die Spaltung dieser Bindung (Ablösung vom Harz) am Ende der Synthese geschieht mit 1-%iger Trifluoressigsäure in Methylenchlorid. Aber auch diese Methode, die wahrscheinlich als eine der besten des Standes der Technik betrachtet werden kann, ist nicht allgemein anwendbar, da gemäss der Feststellung der Autoren selber unter den Bedingungen der Abspaltung am Ende der Synthese mindestens 2 sehr wichtige Schutzgruppen der Seitenketten, und zwar die tert-Butoxycarbonyl-Schutzgruppe der ε-Aminogruppe von Lysin und die tert-Butylgruppe als Schutz der Hydroxylgruppe von Tyrosin, so leicht abgespalten werden, dass die Anwendung dieser Schutzgruppen in Frage gestellt ist. Ueberdies ist das Harz für eine direkte Festphase-Synthese von Peptidamiden ungeeignet. Dies alles ist wahrscheinlich ein Grund dafür, dass diese Methode trotz ihrer offensichtlichen Vorteile bisher keine breitere Anwendung gefunden hat.

Ueberraschenderweise wurde nun gefunden, dass das erfindungsgemässe Kunstharz frei von den bekannten Nachteilen der früheren Lösungen ist und es somit ermöglicht, die Festphase-Synthese von geschützten und ungeschützten Peptiden und Peptidamiden breit anzuwenden. Als Polymer-Gerüst wird vorzugsweise das meistgebrauchte Trägerpolymer, d.h. Polystyrol, zu seinem Aufbau benutzt.

Als Grundpoplymer ist praktisch jedes Kunstharz, das Phenylgruppen im Gerüst enthält, anwendbar. Bevorzugt sind Polymere von Styrol, wie sie seit 20 Jahren als Träger für die Festphase-Synthese von Peptiden im allgemeinen Gebrauch sind und in Form verschiedener kommerziellen Präparate zu diesem Zweck erhältlich sind. Zwecks Erhöhung der Stabilität und Unlöslichkeit in organischen Lösungsmitteln werden Polystyrolharze bevorzugt, die durch Copolymerisieren mit höchstens 5, vorzugsweise etwa 1-2 Mol% Divinylbenzol quervernetzt sind. Solche Grundpolymere werden durch Chlormethylierung oder Brommethylierung an ihren Phenylgruppen (Benzolringen) substituiert und dann die eigentlichen Ankergruppen durch Austausch von Chlor bzw. Brom an der Methylengruppe eingeführt. Auch halogenmethylierte, vor allem chloromethylierte Polystyrolharze sind geläufige kommerzielle Produkte, die unter der Bezeichnung "Merrifield-Harz" eine breite Anwendung in der Festphase-Synthese von Peptiden finden.

Das erfindungsgemässe Verfahren zur Herstellung der oben definierten neuartigen Kunstharze besteht darin, dass man ein geeignetes, z.B. ein oben beschriebenes, mit 0-5% Divinylbenzol quervernetztes und an Benzolringen des Grundgerüsts chlormethyliertes oder brommethyliertes Polystyrol nacheinander
a) mit einer Verbindung der Formel 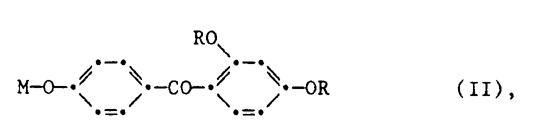 worin M ein Alkalimetall ist und R die obgenannte Bedeutung hat,
b) mit einem Reduktionsmittel, und, falls X für -NH- steht,
c) mit einem die Aminogruppe einführenden Reagens umsetzt.

Die Umsetzung gemäss Verfahrensstufe a) erfolgt in Gegenwart eines hochpolaren Lösungsmittels, welches vorzugsweise gutes Lösungsvermögen für Salze hat, beispielsweise eines dipolar aprotischen Lösungsmittels, wie Dimethylsulfoxid, Acetonitril, Hexamethylphosphortriamid, N,Nʹ-Propylenharnstoff oder eines aliphatischen Amids, wie insbesondere Dimethylformamid, ferner auch Mischungen der genannten Lösungsmittel untereinander, und vorzugsweise unter strengem Ausschluss von Feuchtigkeit. Vornehmlich wird mit einer Lösung des Alkalimetallsalzes der Formel II gearbeitet; zu diesem Zweck ist ein Cäsiumsalz (M ist Cäsium) besonders gut geeignet. Die Reaktion kann bei Temperaturen zwischen 0-50°C, vorzugsweise in der Umgebung der Raumtemperatur, durchgeführt werden, wobei die Reaktionszeiten sich entsprechend auf mehrere, z.B. 8-72 Stunden erstrecken. Das Reaktionsgemisch wird dabei vorzugsweise mechanisch gerührt oder geschüttelt. Die als Ausgangsstoff verwendeten 2,4-Dialkoxy-4ʹ-hydroxybenzophenone sind in an sich bekannter Weise, z.B. durch C-Acylierung eines entsprechenden Resorcin-diethers mit p-Hydroxybenzoylchlorid unter Katalyse mit Aluminiumchlorid, oder durch eine Modifikation dieser Acylierung, zugänglich; das gewünschte Salz wird daraus durch konventionelles Umsetzen mit dem entsprechenden Alkalimetallhydroxid, wie Cäsiumhydroxid, erhalten.

Die Verfahrensstufe b) wird in an sich bekannter Weise durch Umsetzen mit einem Reduktionsmittel, z.B. einem solchen, das zur Reduktion von Oxogruppen zu Hydroxylgruppen gebräuchlich ist, durchgeführt. Geeignete Reduktionsmittel sind z.B. Diboran oder komplexe Hydride, wie insbesondere Alkalimetall-borhydride (z.B. Natriumborhydrid, Lithiumborhydrid oder Kaliumborhydrid) sowie auch Alkalimetall-aluminiumhydride (z.B. Natriumaluminiumhydrid oder Lithiumaluminiumhydrid), welche in zweckmässigen nicht-reaktiven organischen Lösungsmitteln, insbesondere offenkettigen oder cyclischen Ethern (z.B. Diisopropylether oder 1,2-Dimethoxy- oder Diethoxy-ethan bzw. Dioxan oder Tetrahydrofuran) bei Temperaturen von 0 bis etwa 100°C, abhängig vom Reagens, eingesetzt werden. Die Reaktionszeiten sind von den jeweiligen Reagentien und Reaktionsbedingungen abhängig, in der Regel bewegen sie sich zwischen 1-48 Stunden; Umschütteln oder Rühren erleichtert den Kontakt der festen und flüssigen Komponenten. Wenn notwendig, kann der Reduktionsvorgang mit einer neuen Portion Reduktionsmittel wiederholt werden; überschüssiges Reagens wird am Ende der Reaktion vorteilhaft zerstört, z.B. durch ein Keton, wie Aceton.

Die gewünschtenfalls durchzuführende Verfahrensstufe c), die zur Ueberführung des gemäss b) erhaltenen "Hydroxylharzes" in das "Aminoharz" dient, erfolgt z.B. mit Ammoniak. Dazu wird beispielsweise Ammoniakgas in eine gerührte Suspension des "Hydroxylharzes" in einem polaren Lösungsmittel, z.B. einem bei Verfahrensstufe a) oder b) erwähnten Lösungsmittel bei Temperaturen zwischen 0°C und Raumtemperatur eingeleitet; vornehmlich kann man auch unter erhöhtem Druck und Temperaturen bis 50°C mit Umschütteln arbeiten.

Besonders geeignet ist die Aminogruppe einführende Reagentien für die Verfahrensstufe c) sind Carbamate, deren Alkohol-Teil durch Basenbehandlung abgespalten werden kann, die aber unter sauren Reaktionsbedingungen stabil sind. Beispiele für solche Carbamate sind substituierte Ethylcarbamate, die in der β-Stellung aktivierende, insbesondere elektronenziehende Substituenten tragen. Geeignet sind β-(Niederalkan- oder Arylsulfonyl)-ethylcarbamate, z.B. β-(Methansulfonyl)-ethylcarbamat oder β-(Benzosulfonyl)-ethylcarbamat, β-(Nitro-, Cyano- oder Halogenphenyl)-ethylcarbamate, z.B. β-(p-Nitrophenyl)-ethylcarbamat, β-Di-(p-nitrophenyl)-ethylcarbamat oder β-(Pentafluorophenyl)-ethylcarbamat, oder insbesondere 9-Fluorenylmethylcarbamat. Bei der Reaktion mit den oben genannten Carbamaten wird eine Suspension des "Hydroxylharzes" in einem inerten organischen Lösungsmittel, beispielsweise einem Halogenkohlenwasserstoff, wie Methylenchlorid, Chloroform oder 1,2-Dichlorethan, oder einem Ether, wie Diisopropylether, Diisobutylether, Dimethoxyethan, Diethoxyethan, Tetrahydrofuran oder Dioxan, bei Temperaturen zwischen 20° und 80°C, vorzugsweise um 50°C, mit dem Carbamat und einer starken Säure, beispielsweise einer organischen Sulfonsäure, vorzugsweise einer Niederalkan- oder Arylsulfonsäure, z.B. Methansulfonsäure, Benzolsulfonsäure oder p-Toluolsulfonsäure während einigen Stunden, z.B. 2 bis 44 Stunden, gerührt. Auf diese Weise entsteht ein Kunstharz der oben definierten Struktur, welches anstelle der Gruppe -X-H eine Gruppe -NH-W trägt, worin W eine durch Basenbehandlung abspaltbare Aminoschutzgruppe, insbesondere eine substituierte Ethoxycarbonylgruppe, bedeutet. Zur Freisetzung des "Aminoharzes" wird danach die Schutzgruppe mit Base abgespalten, beispielsweise mit einer Lösung eines tertiären oder vorzugsweise sekundären offenkettigen oder cyclischen Amins, z.B. Triethylamin, Tributylamin, Diethylamin, Piperidin, Pyrrolidin oder Morpholin, in einem inerten organischen Lösungsmittel, vorzugsweise in einem der obengenannten Ether oder in einem Diniederalkylamid, z.B. Dimethylformamid oder Dimethylacetamid, bei Temperaturen zwischen 0° und 50°C, vorzugsweise um Raumtemperatur, mit Reaktionszeiten von wenigen Minuten, z.B. 1 Minute, bis zu einigen Stunden, z.B. 6 Stunden. Anstelle eines Amins kann auch ein Alkalimetallhydroxid, z.B. Natriumhydroxid, oder ein Ammoniumhydroxid, z.B. Benzyltrimethylammoniumhydroxid, eingesetzt werden, wobei die Spaltung auch bei tieferen Temperaturen schon nach kürzeren Reaktionszeiten, z.B. nach weniger als 1 Minute, abgeschlossen ist.

Wie bereits oben erwähnt, betrifft die Erfindung auch die Verwendung des erfindungsgemässen Kunstharzes als Träger für die Festphase-Synthese von Peptiden und Peptidamiden, insbesondere von solchen, die an der N-terminalen Aminogruppe und/oder an funktionellen Gruppen der Seitenketten geschützt sind.

Erfindungsgemäss werden diese peptidischen Verbindungen unter Verwendung des oben definierten Kunstharzes hergestellt, indem man
a) das Kunstharz mit einer Verbindung der Formel W¹-AM¹-Y-H, worin Y für -O- oder -NH-, W¹ für eine N-terminale Aminoschutzgruppe und AM¹ für den Acylrest einer an funktionellen Gruppen gewünschtenfalls geschützten Aminosäuresequenz bestehend aus 1-25 Aminosäureresten steht, oder mit einem reaktionsfähigen funktionellen Derivat davon zwecks Verankerung des Rests W¹-AM¹- an der Gruppe -X- des Harzes umsetzt,
b) die N-terminale Schutzgruppe W¹ abspaltet,
c) die freigewordene N-terminale Aminogruppe durch Umsetzen mit einer Säure der Formel W²-AM²-OH, worin W² und AM² analoge Bedeutung wie die oben definierten Reste W¹ und AM¹ haben, oder mit einem reaktionsfähigen funktionellen Derivat davon acyliert,
d) den Vorgang der wechselweisen Abspaltung gemäss b) und Acylierung gemäss c) beliebige Male bis zum Erreichen einer gewünschten Aminosäuresequenz wiederholt und
e) das gebildete Peptid oder Peptidamid, gewünschtenfalls nach vorangehender oder mit gleichzeitiger Abspaltung von Schutzgruppen, durch Acidolyse vom Harz abspaltet.

Bei der Verankerung der C-terminalen geschützten Aminosäure oder Aminosäuresquenz am erfindungsgemässen Harz werden die Bedingungen immer mit Rücksicht auf die jeweiligen reagierenden funktionellen Gruppen gewählt. Vorzugsweise verwendet man als Trägerharz das "Hydroxylharz" der Formel I, worin X für -O- steht; dieses kann man, falls der Endstoff in der Amidform erwünscht ist, mit einem geschützten Aminosäureamid oder einer geschützten Aminosäuresequenz, welche als Amid vorliegt, z.B. mit einer Verbindung der Formel W¹-AM¹-NH₂, worin W¹ und AM¹ die oben definierten Bedeutungen haben, umsetzen, wobei die Hydroxylgruppen -XH des Harzes gegen die amidische C-terminale Aminogruppe ausgetauscht werden. Die Verankerungsreaktion wird in der Regel under Säurekatalyse, z.B. mittels einer organischen Sulfonsäure, vorzugsweise einer Niederalkan- oder Arylsulfonsäure, z.B. Methansulfonsäure, Benzolsulfonsäure oder p-Toluolsulfonsäure, in Gegenwart von inerten organischen Lösungsmitteln, wie chlorierten Alkanen (z.B. Methylenchlorid, Chloroform oder 1,2-Dichlorethan) und/oder offenkettigen oder cyclischen Ethern (z.B. Diethyl-, Diisopropyl- oder Dibutylether, oder 1,2-Dimethoxy- oder 1,2-Diethoxyethan, bzw. Dioxan oder Tetrahydrofuran) während mehreren, z.B. 2-48 Stunden bei Temperaturen von 20°-80°C, vorzugsweise bei etwa 50°C, durchgeführt. Wenn notwendig kann die Verankerungsoperation wiederholt werden. Das Harz mit verankertem C-terminalen Amid, d.h. ein solches, dessen Benzolringe mit den Resten der Formel 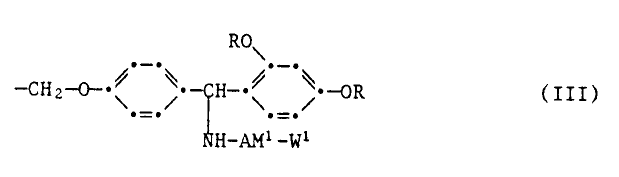
worin R, W¹ und AM¹ die obengenannte Bedeutungen haben, substituiert ist, wird dann dem Aufbau der gewünschten Aminosäuresequenz gemäss den Verfahrensstufen b)-d) unterworfen. Die Ablösung des fertigen Peptidamids (Verfahrensstufe e) kann mit Fluorwasserstoff, vorzugsweise aber mit milderen sauren Mitteln, wie mit Trifluoressigsäure, vorzugsweise in einem inerten organischen Verdünnungsmittel, wie einem Halogenalkan, oder in Wasser, vorgenommen werden. Beispielsweise wird die Ablösung mit einem Gemisch von Trifluoressigsäure mit Methylenchlorid oder 1,2-Dichlorethan, z.B. im Volumenverhältnis 1:1, vorgenommen. Unter diesen Bedingungen sind jedoch nicht alle üblich verwendeten acidolytisch abspaltbaren Schutzgruppen beständig. Je nach Reaktionsführung werden deshalb gleichzeitig mit der Ablösung vom Harz auch die entsprechenden Schutzgruppen abgespalten und es resultiert ein Peptidamid mit freien funktionellen Gruppen.

Soll das Peptid am Ende der Synthese in Form einer freien Säure (gewünschtenfalls mit einer N-terminalen Schutzgruppe und/oder sonstigen Schutzgruppen) vorliegen, so verwendet man als Trägerharz wiederum das "Hydroxylharz" der Formel I, worin X für -O- steht. Dieses wird dann mit einer geschützten Aminosäure oder mit einer geschützten Aminosäuresequenz in Form der freien Säure oder in Form eines reaktiven funktionellen Derivats der Säure, z.B. mit einer Verbindung der Formel W¹-AM¹-OH, worin W¹ und AM¹ die oben definierten Bedeutungen haben, umgesetzt, wobei es zur Veresterung der Hydroxylgruppe des Harzes kommt. Die Verankerungsreaktion wird unter den in der Peptidsynthese üblichen Bedingungen der Acylierung durchgeführt. Als reaktives funktionelles Derivat verwendet man z.B. ein Anydrid der zu verankernden Säure, insbesondere ein symmetrisches Anydrid, vorzugsweise in Anwesenheit von organischen tertiären Basen, wie den nachfolgend genannten. Ein geeignetes funktionelles Derivat ist auch ein Aktivester der zu verankernden Säure, welcher ebenfalls in Anwesenheit der nachfolgend genannten organischen tertiären Basen umgesetzt wird. Geeignete Aktivester sind insbesondere Ester von Phenolen, die elektronenziehende Substituenten tragen, und Ester von N-Hydroxyimiden. Vorzugsweise verwendet man die zu verankernde Verbindung aber als freie Säure und acyliert mit Hilfe von Carbodiimiden, z.B. Diisopropylcarbodiimid oder insbesondere Dicyclohexylcarbodiimid, als Kondensationsvermittler. Die Reaktion wird in Gegenwart von organischen Basen durchgeführt, insbesondere von tertiären Basen (wie Pyridin, Chinolin, 4-Dimethylaminopyridin, N-Methylpiperidin, N-Methylmorpholin, N,Nʹ-Dimethylpiperazin bzw. Triethylamin oder Diisopropylethylamin) und in inerten organischen Lösungsmitteln, wie chlorierten Alkanen (z.B. Methylenchlorid oder Chloroform) und/oder offenkettigen oder cyclischen Ethern (z.B. Diethyl-, Diisopropyl-oder Dibutylether, oder 1,2-Dimethoxy- oder 1,2-Diethoxyethan, bzw. Dioxan oder Tetrahydrofuran). Vorzugsweise wird eine aktivierte N-Hydroxyverbindung zugesetzt, z.B. 1-Hydroxybenztriazol. Die Reaktionszeit beträgt in der Regel mehrere, wie 2-48, Stunden bei Temperaturen von 0°-40°C; gewünschtenfalls kann der Acylierungsvorgang wiederholt werden. Als eine Massname zur Vermeidung von unerwünschten Nebenprodukten ist es vorteilhaft, das erhaltene Harz mit Benzoylchlorid (oder einem ähnlichen einfachen Säurederivat) in Gegenwart einer Base, wie einer oben erwähnten, zu behandeln und somit die gegebenenfalls noch freigebliebenen Hydroxylgruppen des Harzes zu blockieren. Das Harz mit verankertem C-terminalen Ester, d.h. ein solches, dessen Benzolringe mit den Resten der Formel 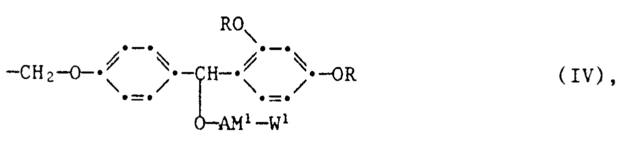
worin R, W¹ und AM¹ die obengenannte Bedeutungen haben, substituiert ist, wird dann dem Aufbau der gewünschten Aminosäuresequenz gemäss den Verfahrensstufen b)-d) unterworfen. Die Ablösung der fertigen Peptidsäure (Verfahrensstufe e) kann, falls ein Endstoff ohne Schutzgruppen, insbesondere ohne säure-labile Schutzgruppen erwünscht ist, mit Fluorwasserstoff, vorzugsweise aber mit milderen sauren Mitteln, wie Trifluoressigsäure, die vorzugsweise verdünnt wird mit einem inerten organischen Lösungsmittel, wie einem Halogenalkan, durchgeführt werden. Falls ein Peptid (Aminosäuresequenz) erwünscht ist, worin die N-terminale Aminogruppe und/oder die sonstigen funktionellen Gruppen in geschützter Form erhalten bleiben, wie es bei Peptidfragmenten für weitere Synthese meist wünschenswert ist, wird die Ablösung des Endprodukts vom Harz unter besonders milden acidolytischen Bedingungen durchgeführt, z.B. mit einer organischen Säure, insbesondere einer Niederalkancarbonsäure, vorzugsweise Ameisen- oder Propionsäure und vor allem Essigsäure, welche mit neutralen organischen Lösungsmitteln, z.B. chlorierten Alkanen oder aliphatischen oder cyclischen Ethern, verdünnt werden kann; als besonders vorteilhaft ist beispielsweise ein Gemisch von Essigsäure und Methylenchlorid oder Chloroform im Volumenverhältnis von etwa 1:1 bis etwa 1:19, insbesondere 1:9, zu erwähnen. Geeignet sind auch stark verdünnte Trifluoressigsäure, z.B. als 0,1 bis 2-%ige Lösung in Methylenchlorid oder Chloroform, oder ein Pyridiniumsalz, z.B. Pyridiniumhydrochlorid, in einem polaren, salz-und peptidlösenden Lösungsmittel, z.B. in Dimethylacetamid oder Dimethylformamid. Die Ablösung erfolgt üblicherweise im Temperaturbereich von 0°-50°C, vorzugsweise in Umgebung von Raumtemperatur, wobei die Reaktionszeit sich von wenigen Minuten bis mehrere (z.B. 2-8) Stunden erstreckt.

Zu Peptidamiden kommt man auch, wenn man als Trägerharz das "Aminoharz" der Formel I, worin X für -NH- steht, mit einer geschützten Aminosäure oder geschützten Aminosäuresequenz in Form der freien Säure oder in Form eines reaktiven funktionellen Derivats der Säure, z.B. mit einer Verbindung der Formel W¹-AM¹-OH, worin W¹ und AM¹ die oben definierten Bedeutungen haben, umsetzt. Dabei ensteht ein Harz mit verankertem C-terminalem Amid, d.h. ein solches, dessen Benzolringe mit den Resten der Formel III substituiert sind. Die Umsetzung erfolgt vorzugsweise unter den oben bei der Veresterung des "Hydroxylharzes" mit freier Säure oder einem reaktiven funktionellen Derivat davon genannten Reaktionsbedingungen. Der Aufbau der gewünschten Aminosäuresequenz gemäss den Verfahrensstufen b) bis d) und die Ablösung des fertigen Peptidamids gemäss Verfahrensstufe e) erfolgt dann genau wie weiter vorn beschrieben.

Die relativ leichte Abspaltbarkeit der Aminosäuresequenz vom Harz bedingt eine besondere Auswahl der N-terminalen Aminoschutzgruppen, welche streng selektiv unter Erhalt der Verankerung der Sequenz am Harz (und auch unter Erhalt sonstiger Schutzgruppen), aber dennoch quantitativ abspaltbar sein muss. Diese N-terminale Aminoschutzgruppe W¹ bzw. W² ist vorzugsweise eine basisch abspaltbare Gruppe, insbesondere eine vom Oxycarbonyl-Typ. Bevorzugte N-terminale Aminoschutzgruppen W¹ bzw. W² sind substituierte Ethoxycarbonylgruppen, die in der β-Stellung aktivierende, insbesondere elektronenziehende Substituenten tragen, wie sie weiter vorn bei den enstprechenden Carbamaten genannt sind. In erster Linie kommen die β-(Methansulfonyl)-ethoxycarbonylgruppe, die β-(p-Nitrophenyl)-ethoxycarbonylgruppe und die β-Di-(p-Nitrophenyl)-ethoxycarbonylgruppe in Frage, vor allem aber die 9-Fluorenylmethoxycarbonylgruppe (Fmoc). Diese Schutzgruppen können mit anorganischen oder organischen Basen, insbesondere mit tertiären oder sekundären Aminen, wie sie weiter vorn bei der Herstellung von "Aminoharz" genannt sind, abgespalten werden. Die Bedingungen der Abspaltung der N-terminalen Aminoschutzgruppe gemäss Verfahrensstufe b), insbesondere der Fmoc-Gruppe, sind weiter vorn bei der Herstellung von "Aminoharz" beschrieben und allgemein bekannt.

Das Acylieren der freigesetzten N-terminalen Aminogruppe gemäss Verfahrensstufe c) erfolgt unter den allgemein üblichen Bedingungen, die für die Festphase-Synthese, insbesondere am Merrifield-Träger, geläufig sind. Unter zahlreichen bekannten Varianten ist diejenige hervorzuheben, bei welcher man mit einer am N-Terminus mit Fmoc oder mit einer äquivalenten basenlabilen Schutzgruppe geschützten Aminosäure (oder Aminosäuresequenz), worin alle funktionellen Gruppen der Seitenkette in geschützter Form vorliegen, oder mit einem reaktiven funktionellen Derivat dieser Säure, z.B. dem symmetrischen Anhydrid oder einem Aktivester, acyliert. Verwendet man die freie Säure, so wird als Kondensationsmittel Dicyclohexylcarbodiimid (oder ein analoges Reagens), vorzugsweise in Kombination mit 1-Hydroxybenztriazol, und in Gegenwart von tertiären organischen Basen, z.B. tertiären aliphatischen oder cyclischen Aminen oder heteroaromatischen Basen, wie Triethylamin, Diisopropylethylamin, N,N-Dimethylanilin, N-Methylpiperidin, N-Ethylpiperidin, N-Methylmorpholin, N,Nʹ-Dimethylpiperazin bzw. Pyridin und dessen Homologen, Chinolin oder 4-Dimethylaminopyridin, eingesetzt. Auch die Acylierung mit dem symmetrischen Anhydrid oder dem Aktivester erfolgt in Gegenwart der genannten tertiären Basen und vorzugsweise auch von 1-Hydroxybenztriazol. Als Aktivester kommen Ester mit Phenolen, die elektronenziehende Substituenten tragen, z.B. mit p-Nitrophenol, Pentafluorophenol oder 2,4,5-Trichlorphenol, oder mit N-Hydroxyimiden, z.B. mit N-Hydroxysuccinimid oder N-Hydroxynorbornan- oder 5-norbornen-2,3-dicarbonsäureimid, in Frage. Die Acylierungsbedingungen der genannten Varianten sind allgemein bekannt; den Acylierungsvorgang kann man gewünschtenfalls wiederholen und/oder, um Bildung von falschen Sequenzen zu verhindern, den restlichen nichtacylierten Ausgangsstoff mit einem einfachen Carbonsäurederivat, wie Essigsäureanhydrid oder -chlorid, in einer konventionellen Weise acylieren und damit gegen weitere unerwünschte Acylierungen durch Aminosäurereste in späteren Synthesestufen blockieren.

Als Aminosäurereste in der erfindungsgemässen Synthese kommen in erster Linie diejenigen in Betracht, die sich von in der Natur, insbesondere als Peptid-Bausteine, vorkommenden α-Aminosäuren der L-Reihe und deren nahe verwandten Analogen, wie insbesondere den Enantiomeren der "unnatürlichen" D-Reihe, ableiten. Bevorzugte α-Aminosäuren sind beispielsweise Glycin, Alanin, Valin, Leucin, Isoleucin, Phenylalanin, Asparaginsäure, Glutaminsäure, Arginin, Histidin und Lysin, ferner α-Aminobuttersäure, Norvalin, Isovalin, Norleucin, Ornithin und Citrullin, sowie auch Asparagin, Glutamin, Tyrosin, Tryptophan, Methionin, Threonin, Serin, aber auch Prolin und Hydroxyprolin (bei denen die α-Aminogruppe mit dem Alkylrest zu einem Ring zusammengeschlossen ist), sowie Cystein und Cystin (wobei dieses als ein Paar von 2 zusammengebundenen Cysteinresten, die sich auch auf voneinander getrennten Positionen der Sequenz befinden können, vorkommt). Ferner kommen auch Reste von anderen Aminosäuren in Betracht, die sich von C₁-₇-Alkancarbonsäuren, insbesondere linearen, ableiten und die Aminogruppe in einer beliebigen Stellung der Kette tragen, beispielsweise am endständigen C-Atom (wie in β-Alanin, γ-Aminobuttersäure oder δ-Aminovaleriansäure); zusätzlich können sie auch weitere primäre Aminogruppen tragen (wie in der α,γ-Diaminobuttersäure) oder durch andere funktionelle Gruppen, wie Hydroxyl-, Mercapto-, Disulfido-, Guanidino-, Carboxyl- oder Carboxamidogruppen, oder durch cyclische Hydrocarbyl- oder Heterocyclylreste, wie Phenyl, p-Hydroxyphenyl, Indolyl oder Imidazolyl, substituiert sein. Diese Aminosäuren können, soweit sie asymmetrische C-Atome enthalten, in racemischer oder, vorzugsweise, in optisch aktiver Form eingesetzt werden.

Der spezifische Charakter der Festphase-Synthese verlangt, dass in den eingesetzten Aminosäuresten nicht an der Reaktion teilnehmende funktionelle Gruppen (und zwar auch solche, die bei Synthesen in flüssiger Phase frei bleiben können) in der Regel in einer geschützten Form vorliegen.

Die Auswahl der Schutzgruppen richtet sich nach den Synthesebedingungen und nach der Verwendung des herzustellenden Endstoffs. Bei mehreren zu schützenden funktionellen Gruppen muss man zweckmässige Kombinationen wählen. Insbesondere ist darauf zu achten, dass Schutzgruppen von funktionellen Gruppen der Aminosäure-Seitenketten bei der Abspaltung der N-terminalen Schutzgruppe, wie der Fmoc-Gruppe, beständig sind, und, falls als Endstoff ein geschützter Peptidbaustein für eine weitere Synthese erwünscht ist, dass sie bei den milden sauren Bedingungen der Ablösung vom Harz am Ende der Synthese stabil sind.

Zum Schutz anderer vorhandener Aminogruppen, wie der ε-Aminogruppe im Lysinrest, kann jede in der Peptid-Chemie übliche Amino-Schutzgruppe verwendet werden, die unter schwach basischen Bedingungen stabil ist. Geeignete Gruppen sind in bekannten Nachschlagewerken, z.B. in Houben-Weyl; Methoden der organischen Chemie, 4.Auflage, Band 15/I, E.Wünsch (Herausgeber), Synthese von Peptiden (Georg Thieme Verlag, Stuttgart, 1974), zusammenfassend beschrieben.

So kann man z.B. reduktiv oder unter energischen basischen Bedingungen abspaltbare Amino-Schutzgruppen verwenden, z.B. insbesondere die Benzyloxycarbonyl-Gruppe und Benzyloxycarbonyl-Gruppen, die im aromatischen Teil durch Halogenatome, Nitrogruppen, Niederalkoxygruppen und/oder Niederalkylreste substituiert sind, wie die p-Chlor- oder p-Brombenzyloxycarbonyl-, p-Nitrobenzyloxycarbonyl-, p-Methoxybenzyloxycarbonyl- oder p-Tolyloxycarbonyl-Gruppe, ferner die Isonicotinyloxycarbonylgruppe, weiter auch Sulfonylgruppen, wie p-Toluolsulfonyl, Benzolsulfonyl, o-Nitrobenzolsulfonyl und andere substituierte Benzolsulfonylgruppen bzw. auch Acylgruppen, wie Formyl, Trifluoracetyl oder Phthaloyl. Eine vorteilhafte Amino-Schutzgruppe ist eine Ethoxycarbonylgruppe, die in β-Stellung eine mit drei Kohlenwasserstoffresten substituierte Silylgruppe, wie Triphenylsilyl-, Dimethyl-tert-butyl-silyl- oder vor allem Trimethylsilylgruppe, trägt. Eine solche β-(Trihydrocarbylsilyl)-ethoxycarbonylgruppe, wie eine β-(Triniederalkylsilyl)-ethoxycarbonyl-, z.B. insbesondere die β-(Trimethylsilyl)-ethoxycarbonylgruppe, ist unter den Bedingungen der sauren Hydrolyse und der Hydrogenolyse beständig, kann aber unter ganz spezifischen, sehr milden Bedingungen durch Einwirkung von Fluoridionen abgespalten werden. In dieser Beziehung verhält sie sich analog wie die weiter unten als Carboxyl-Schutzgruppe beschriebene β-Silylethylestergruppe.

Ganz besonders bevorzugt sind acidolytisch abspaltbare Gruppen, wie in erster Linie die tert-Butoxycarbonyl-Gruppe und analoge Gruppen, z.B. die tert-Amyloxycarbonyl-, Isopropyloxycarbonyl-, Diisopropylmethoxycarbonyl-, Allyloxycarbonyl-, Cyclopentyloxycarbonyl-, Cyclohexyloxycarbonyl-, d-Isobornyloxycarbonyl- und Adamantyloxycarbonylgruppen, sowie auch Gruppen des Aralkyl-Typs, wie Benzhydryl und Triphenylmethyl (Trityl), ferner Aralkoxycarbonyl-Gruppen des 2-Aryl-2-propyloxycarbonyl-Typs, z.B. die 2-Phenyl- oder 2-p-Biphenylyl-2-propyloxycarbonylgruppen.

Als Schutzgruppe der Guanidino-Funktion, wie sie beispielsweise in der natürlichen Aminosäure Arginin vorkommt, kann eine der obengenannten Aminoschutzgruppen verwendet werden. Besonders geeignet sind Sulfonylgruppen, insbesondere Niederalkan-, z.B. Methan-, Ethan-oder Isopropansulfonylgruppen, oder Aryl-, z.B. substituierte Benzolsulfonylgruppen, wobei als Substituenten Niederalkyl, z.B. Methyl, Niederalkoxy, z.B. Methoxy, annelliertes Niederalkoxy, z.B. 1-Oxa-1,4-butylen, Nitro oder Halogen, z.B. Chlor oder Brom, in Frage kommen. Besonders bevorzugt ist die 2,3,5-Trimethyl-4-methoxybenzolsulfonylgruppe und die 2,2,5,7,8-Pentamethyl-chroman-6-sulfonylgruppe.

Als Hydroxyl-Schutzgruppe können alle zu diesem Zweck in der Peptid-Chemie gebräuchlichen Gruppen verwendet werden, vgl. den oben zitierten Uebersichtsartikel in Houben-Weyl. Bevorzugt sind acidolytisch abspaltbare Gruppen, wie 2-Tetrahydropyranyl und ganz besonders tert-Butyl, sowie auch tert-Butoxycarbonyl. Ferner kann man aber auch reduktiv oder basisch abspaltbare Hydroxyl-Schutzgruppen verwenden, z.B. Benzyl- oder Benzyloxycarbonylgruppen, die im aromatischen Teil durch Halogen, Nitro und/oder Niederalkoxy substituiert sein können, Niederalkanoylreste, wie Acetyl, oder Aroylreste, wie Benzoyl.

Als Carboxyl-Schutzgruppe kann jede übliche zu diesem Zwecke gebräuchliche Gruppe verwendet werden, vgl. den oben zitierten Uebersichtsartikel in Houben-Weyl. So werden Carboxylgruppen beispielsweise durch Hydrazidbildung oder durch Veresterung geschützt. Zur Veresterung geeignet sind z.B. niedere, gegebenenfalls substituierte Alkanole, wie Methanol, Ethanol, Cyanmethylalkohol, 2,2,2-Trichlorethanol, Benzoylmethylalkohol oder insbesondere tert-Butylalkohol, aber auch ein gegebenenfalls substituierter Benzylalkohol. Eine besonders vorteilhafte Kategorie der substituierten Alkanole sind Ethylalkohole, die in β-Stellung eine trisubstituierte Silylgruppe, wie Triphenylsilyl-, Dimethyl-tert-butyl-silyl- oder vor allem Trimethylsilylgruppe, tragen. Diese Alkohole eignen sich zum Schutze der Carboxylgruppen deshalb besonders gut, weil die entsprechenden β-Silylethylester, z.B. β-(Trimethylsilyl)ethylester, zwar die Stabilität üblicher Alkylester besitzen, sich jedoch unter Erhalt aller anderen Schutzgruppen mit Fluoridionen selektiv abspalten lassen.

Als Mercapto-Schutzgruppe kann man alle zu diesem Zweck in der Peptid-Chemie gebräuchlichen Gruppen verwenden. Die Mercaptogruppen werden insbesondere durch geeignete Acylierung oder Alkylierung geschützt. Zur Acylierung geeignet ist z.B. der Acetyl- oder Benzoylrest, eine Niederalkyl- (z.B. Ethyl-) -carbamoyl- oder eine gegebenenfalls, wie oben angegeben, substituierte Benzyloxycarbonyl-Gruppe. Zur Alkylierung geeignet sind z.B. der tert-Butyl-, Isobutyloxymethyl-, Benzylthiomethyl- oder Tetrahydropyranylrest oder gegebenenfalls durch Halogen, Niederalkoxy oder Nitro substituierte Arylmethylreste wie Benzyl, p-Methoxybenzyl, Diphenylmethyl, Dimethoxybenzhydryl oder ganz besonders Trityl, sowie auch Phenylcyclohexyl, p-Methoxyphenylcyclohexyl, oder 2-Thienylcyclohexyl. Sehr vorteilhaft ist auch ein Acylaminomethylrest, worin Acyl z.B. Acetyl oder auch Benzoyl ist. Besonders bevorzugt ist die Acetylaminomethylgruppe.

Vorzugsweise werden die Schutzgruppen der Seitenketten so gewählt, dass sie alle unter ähnlichen Bedingungen abspaltbar sind; besonders bevorzugt sind dabei die bereits hervorgehobenen acidolytisch abspaltbaren Gruppen, vorallem die vom tert-Butyl abgeleiteten. Die Abspaltung aller dieser Schutzgruppen erfolgt dann vorteilhaft in einer einzigen Operation.

Die Abspaltung der Schutzgruppen erfolgt in der allgemein bekannten Weise. Die Acidolyse bzw. saure Hydrolyse wird z.B. mittels Trifluoressigsäure, Salzsäure oder Fluorwasserstoff, bei leicht spaltbaren Schutzgruppen auch mittels einer niederaliphatischen Carbonsäure, wie Ameisensäure und/oder Essigsäure, in Anwesenheit eines halogenierten Kohlenwasserstoffs, z.B. Methylenchlorid, oder von Wasser und gegebenenfalls einem polyhalogenierten Niederalkanol oder Niederalkanon, wie 1,1,1,3,3,3-Hexafluorpropan-2-ol oder Hexafluoraceton, durchgeführt. Die reduktiv abspaltbaren Gruppen, insbesondere solche, die Benzylreste enthalten, werden vorzugsweise hydrogenolytisch, z.B. durch Hydrierung unter Palladium-Katalyse, entfernt. Die Isonicotinyloxycarbonylgruppe wird vorzugsweise durch Zink-Reduktion abgespalten. Die β-Silylethyloxycarbonylgruppe wird durch Fluoridionen gespalten.

Die Erfindung betrifft auch die Zwischenprodukte und Endprodukte des synthetischen Verfahrens unter Verwendung des erfindungsgemässen Harzes. Insbesondere betrifft die Erfindung ein Kunstharz der eingangs definierten Struktur, welches anstelle der Gruppe -X-H die Gruppe -NH-W, -X-AM-W oder -X-AM-H trägt, worin -X- für -O- oder -NH-, W für eine N-terminale Aminoschutzgruppe und AM für den Acylrest einer an funktionellen Gruppen gewünschtenfalls geschützten Aminosäuresequenz bestehend aus 1-180 Aminosäurereste steht, vorallem eine in den nachfolgenden Beispielen beschriebene Verbindung. Ebenfalls betrifft die Erfindung Peptide und Peptidamide der Formel W-AM⁰-XH, worin -X- für -O- oder -NH-, W für eine terminale Aminoschutzgruppe und AM⁰ für den Acylrest einer an funktionellen Gruppen gewünschtenfalls geschützten Aminosäuresequenz bestehend aus 2-180 Aminosäurereste steht, sowie analoge Verbindungen mit freier N-terminaler Aminogruppe der Formel H-AM⁰-XH, sofern sie durch das erfindungsgemässe synthetische Verfahren erhältlich sind, insbesondere die in den nachfolgenden Beispielen beschriebenen Peptide und Peptidamide.

Als Abkürzungen, z.B. zur Bezeichnung von Aminosäuren, Peptiden, Schutzgruppen etc., werden die üblichen, z.B. die im oben zitierten Uebersichtsartikel in Houben-Weyl zusammengestellten Kurzbezeichnungen verwendet. Wenn nicht anders angegeben, beziehen sich die Namen und Kurzbezeichnungen der Aminosäurereste auf Reste der α-Aminosäuren der in der Natur vorkommenden L-Reihe. Wenn nicht anders angegeben, bezeichnet der Ausdruck "nieder", wo immer er im Zusammenhang mit einem organischen Rest oder Verbindung vorkommt, einen solchen Rest oder Verbindung mit höchstens 7, vorzugsweise aber mit höchstens 4 Kohlenstoffatomen.

In den nachfolgenden Beispielen wird die Erfindung weiter illustriert. Die verwendeten Abkürzungen haben die folgende Bedeutung:
- Boc: - tert-Butoxycarbonyl
- But: - tert-Butyl (im Ether)
- DC: - Dünnschichtchromatographie
- DCCI: - Dicyclohexylcarbodiimid
- DMA: - Dimethylacetamid
- DMF: - Dimethylformamid
- DMSO: - Dimethylsulfoxid
- EDC: - 1,2-Ethandichlorid
- FAB-MS: - Fast Atom Bombardment Massenspektrum
- Fmoc: - 9-Fluorenylmethoxycarbonyl
- HOBt: - 1-Hydroxybenztriazol
- HPLC: - Hochdruck-Flüssigkeitschromatographie = high pressure liquid chromatography
- IGF-2: - Insulin-like Growth Factor-2
- MIF: - Macrophage migration Inhibition Factor
- Mtr: - 2,3,5-Trimethyl-4-methoxy-benzolsulfonyl
- OBut: - tert-Butyl (im Ester)
- TFA: - Trifluoressigsäure
- THF: - Tetrahydrofuran
- Trt: - Trityl

### Beispiel 1: 2,4-Dimethoxy-4ʹ-hydroxy-benzophenon (1)

Die Titelverbindung wird analog zur Darstellung von 2,4ʹ-Dihydroxy-4-methoxy-benzophenon, siehe Ray,S., Grover,P.K. und Anand Nitya: Indian Journal of Chemistry 9, 619-623 (1971), aus Resorcin-dimethylether und 4-Hydroxy-benzoesäure synthetisiert. Elementaranalyse, ¹H-NMR- und IR-Spektrum bestätigen die Struktur von *(1)*.

### Beispiel 2: Cäsiumsalz von 2,4-Dimethoxy-4ʹ-hydroxy-benzophenon (1A)

5.00 g *(1)* (19.3 mMol) werden in 40 ml Ethanol und 16 ml Wasser angeschlämmt und eine Lösung von 3.25 g (19 mMol) Cäsiumhydroxidmonohydrat in 4.5 ml Wasser zugefügt. Die Lösung wird im Vakuum konzentriert, in 40 ml Wasser/tert-Butylalkohol (1:1) aufgenommen und lyophilisiert. Das leicht gelbliche Lyophilisat, aus 20 ml tert-Butylalkohol kristallisiert, ergibt das Salz *(1A)* als weisses Pulver.

### Beispiel 3: 4-(2,4-Dimethoxybenzoyl)-Phenoxymethyl-polystyrol (1% DVB quervernetzt) (2)

20.0 g Chlormethyl-polystyrol-1%-Divinylbenzol (DVB) (= Merrifield Polymer Fluka, Schweiz; 0.67 mMol Cl/g) (13.4 mMol) werden während 1 Stunde bei 50°C im Hochvakuum getrocknet. 26.1 g *(1A)* (67 mMol) werden dreimal mit je ca. 500 ml Pyridin angeschlämmt und im Hochvakuum eingedampft, der Rückstand in ca. 700 ml trockenem DMF gelöst und im Hochvakuum auf ca. 400 ml eingeengt. Das Harz wird zugefügt und das Reaktionsgemisch unter Feuchtigkeitsausschluss während 20 Stunden bei Raumtemperatur geschüttelt. Das Harz wird filtriert und mit 100 ml Portionen der folgenden Lösungsmittel gewaschen: 3x Isopropylalkohol, 3x DMF, 3x Wasser, 3x DMF, 5x Wasser, 4x Isopropylalkohol. Das Harz wird im Hochvakuum bei 40-45°C bis zur Massenkonstanz getrocknet. Das IR-Spektrum zeigt die erwartete C=O Bande.

### Beispiel 4: 4-(2,4-Dimethoxyphenyl-hydroxy-methyl)-phenoxymethylpolystyrol [Dimethoxybenzhydryloxymethyl-polystyrol, "Hydroxylharz"] (3)

Eine Aufschlämmung von 7.07 g *(2)* (ca. 4.8 mMol Ketonfunktion) in 50 ml trockenem Tetrahydrofuran (THF) wird mit 14.5 ml 1 M Lithiumborhydrid in THF versetzt und das Reaktionsgemisch während 1 Stunde am Rückfluss unter Feuchtigkeitsausschluss gekocht. Nach dem Abkühlen auf ca. 0-5°C wird das Gemisch mit 24 ml Methanol und, tropfenweise unter gutem Mischung, mit ca. 6 ml Aceton versetzt. Das abfiltrierte Harz wird unter Anwendung von jeweils 30 ml-Portionen des Lösungsmittels wie folgt gewaschen: 3x Methanol, 1x Wasser, 1x wässrige Salzsäure vom pH ca. 3.0, 2x Wasser, 4x Methanol. Das Harz wird im Hochvakuum bei 40-45°C bis zur Massenkonstanz getrocknet. Elementaranalyse: 3.67 % O = 2.31 mMol O/g, IR-Spektrum: keine C=O Bande.

### Beispiel 5: N-[Fmoc-Pro]-4-(2,4-dimethoxyphenyl-amino-methyl)-phenoxymethylpolystyrol [Fmoc-Pro-Aminoharz] (4)

Ein Gemisch von 0.46 g *(3)* (ca. 0.26 mMol), 0.31 g Fmoc-Pro-NH₂ (0.92 mMol), 76.5 µl 1 M Benzolsulfonsäure-Lösung in Dichlormethan (76.5 µMol) und 10 ml Dioxan werden während 3 Stunden und, nach Zugabe von weiteren 76.5 µl 1 M Benzolsulfonsäure-Lösung, noch 20 Stunden bei 50°C gerührt. Das Harz wird filtriert und unter Anwendung von jeweils 5 ml-Portionen des Lösungsmittels wie folgt gewaschen: 5x Methanol, 12x Dichlormethan, 3x Methanol, 3x Dichlormethan. Das Harz wird im Hochvakuum bei 40-45°C bis zur Massenkonstanz getrocknet.
An einer eingewogenen Probe von ca. 20 mg wird die Fmoc-Gruppe durch 4x 2-minütiges Behandeln mit je 300 µl 20% Piperidin und 6x Waschen mit Dimethylacetamid (DMA) abgespalten. Die spektrophotometrische Bestimmung (300 nm) ergibt eine spezifische Beladung von 0.23 mMol Fmoc/g Harz.

### Beispiel 6: N-[Boc-Pro-Glu(OBut)-Ile-Pro]-Aminoharz (5)

0.42 g *(4)* (97 µMol) werden in einem automatischen Peptidsynthesizer mittels des folgenden Prozesses durch nacheinanderfolgendes wechselweises Abspalten der Fmoc-Gruppe und Ankondensieren (Kupplung) von Fmoc-Ile, Fmoc-Glu(OBut) und Boc-Pro mit jeweiligem Blockieren von nicht umgesetzten Aminogruppen durch Acetylierung zu *(5)* umgesetzt.

### Einzeloperationen:

Waschen und Abspalten (Deblockieren) von Fmoc bei Raumtemperatur mit je ca. 10 ml: 1x Isopropylalkohol (1 Min.), 4x DMA entgast (0.5 Min.), 1x Isopropylalkohol (1 Min.), 3x DMA entgast (0.5 Min.), 4x 20% Piperidin in DMA (2 Min.), 2x Wasser-Dioxan (1:1) (1 Min.), 5x DMA entgast (0.5 Min.), 3x DMA dest. (0.5 Min.).
Kupplung: 0.39 mMol N-geschützte Aminosäure, 0.68 ml 0.57 M 1-Hydroxybenztriazol (HOBt) in DMA (0.39 mMol) und 0.18 ml 2.4 M Dicyclohexylcarbodiimid (DCCI) in DMA unter Zusatz von 0.20 ml DMA (20 Min. bei Raumtemperatur, 2 Stunden bei 40°).
Waschen und Acetylieren der restlichen freien Aminogruppen mit: 1x Acetanhydrid-Pyridin-DMA (1:1:8 Vol.) (5 Min.), 3x DMA entgast, 1x Isopropylalkohol (1 Min.), 3x DMA entgast (0.5 Min.).
Das Harz wird mit Isopropylalkohol gewaschen (3x 5 ml) und im Hochvakuum bei Raumtemperatur getrocknet.

### Beispiel 7: H-Pro-Glu-Ile-Pro-NH₂

0.46 g *(5)* (ca. 90 µMol) werden in Trifluoressigsäure-Dichlormethan (1:1 Vol.) während 15 Minuten bei Raumtemperatur gerührt und das Harz abfiltriert und 3x je mit ca. 10 ml Dichlormethan gewaschen. Das Filtrat wird im Vakuum auf ca. 2 ml konzentriert und unter Rühren in 10 ml Ether eingetropft. Die Fällung wird abfiltriert und im Vakuum getrocknet. Das so erhaltene farblose Pulver ist mit authentischem, in Lösung hergestelltem Tetrapeptidamid gemäss Dünnschichtchromatographie und HPLC identisch.

### Beispiel 8: O-[Fmoc-Gly]-Hydroxylharz (6)

2.0 g *(3)* (ca. 1 mMol), 1.10 g Fmoc-Gly-OH (4 mMol) und 0.87 g DCCI (4.2 mMol) werden in 20 ml 1,2-Ethandichlorid (EDC) während 5 Minuten bei 0-5°C gerührt, mit 24 mg 4-Dimethylamino-pyridin (DMAP) (0.2 mMol) und nach weiteren 20 Minuten bei ca. 5°C mit 110 µl N-Methylmorpholin (1 mMol) versetzt. Das Gemisch wird während 4 Stunden bei Raumtemperatur gerührt. Das abfiltrierte Harz wird in einem Peptidsynthesizer mit je 20 ml folgender Lösungsmittel gewaschen: 3x Methanol, 3x EDC, 3x DMA, je 0.5 Min. Zur Blockierung noch vorhandener Hydroxylgruppen des Harzes wird dieses mit 1.23 g Benzoesäureanhydrid (5.4 mMol) in 1.2 ml Pyridin und 6 ml DMA während 2 Stunden bei Raumtemperatur gerührt und dann wie folgt gewaschen: mit je 20 ml 2x Isopropylalkohol, 3x DMA entgast, 2x Isopropylalkohol, 6x EDC, 2x Isopropylalkohol, 3x DMA entgast, 3x Isopropylalkohol, je 0,5 Min. Die im Hochvakuum bei 45°C getrocknete Titelverbindung *(6)* weist einen Fmoc-Gehalt von 0.26 mMol/g auf.

### Beispiel 9: O-[Fmoc-β-Ala]-Hydroxylharz (7)

Die Titelverbindung *(7)* wird analog *(6)* hergestellt und weist einen Fmoc-Gehalt von 0.33 mMol/g auf.

### Beispiel 10: O-[Fmoc-Leu-Pro-Glu(OBut)-Gly-Ser(But)-Pro-Val-Thr(But)-Leu-Asp(OBut)-Leu-Arg(Mtr)-Tyr(But)-Asn-Arg(Mtr)-Val-Arg(Mtr)-Val-β-Ala]-Hydroxylharz (Seitenkettengeschütztes Eglin-Fragment-[β-Ala⁵⁵]-eglin C(37-55)-nonadecapeptid an Hydroxylharz gebunden), (8)

1.10 g *(7)* (0.36 mMol) von Beispiel 9 werden in einem Peptidsynthesizer mit Waschprozessen und Fmoc-Abspaltprozessen analog Beispiel 6 umgesetzt. Die Aminosäuren 10 bis 18 (d.h. 46-54 des Eglins C) werden als 2,4,5-Trichlorphenylester (1.08 mMol) in 1.8 ml DMA unter Zusatz von 1.89 ml 0.57 M HOBt (1.08 mMol) und 0.285 M Diisopropylethylamin (0.54 mMol) in DMA während 2 Stunden bei Raumtemperatur angekuppelt. Bei Aminosäuren 11, 12, 16 und 18 (d.h. 47, 48, 52 und 54 des Eglins C) wird dieser Prozess wiederholt. Die Aminosäuren 1 bis 9 (d.h. 37-45 des Eglins C) werden zur Kupplung als symmetrische Anhydride (1.08 mMol) eingesetzt: 2.16 mMol Fmoc-aminosäure werden in 10 ml Dichlormethan (bei Fmoc-Gly-OH unter Zusatz von 1 ml DMA) gelöst, bei Raumtemperatur mit 245 mg DCCI (1.12 mMol) unter Rühren versetzt, während 15 Minuten bei Raumtemperatur gehalten, der ausgefallene Dicyclohexylharnstoff abfiltriert, zum Filtrat 2.5 ml DMA (dest.) gegeben und das Dichlormethan im Vakuum entfernt. Dieses Gemisch wird jeweils zum Syntheseharz gegeben und nach Zugabe von 58 µl Diisopropylethylamin (0.36 mMol) während 1 Stunde bei Raumtemperatur gehalten. Nach Abschluss der Synthese und Entfernung der endständigen Fmoc-Gruppe wie in Beispiel 5 wird das Harz 3x mit je 5 ml Isopropylalkohol gewaschen und im Hochvakuum bei Raumtemperatur getrocknet.

### Beispiel 11: Seitenkettengeschütztes [β-Ala⁵⁵]-eglin C(37-55)-nonapeptid

1.55 g *(8)* (ca. 83 µMol) von Beispiel 10 werden mit 20 ml Dichlormethan-Essigsäure (9:1 Vol.) während 1.5 Stunden bei Raumtemperatur verrührt, das Harz abfiltriet und mit je 10 ml der Lösungsmittel wie folgt gewaschen: 3x Dichlormethan, 4x Methanol, 4x Dichlormethan. Das ursprüngliche Filtrat wird mit Waschlösungen vereinigt und im Vakuum konzentriert, mit ca. 5 ml Essigsäure versetzt und zur Trockne lyophilisiert, wobei die Titelverbindung als farbloses Pulver zurückbleibt. HPLC an Nucleosil 5C₁₈, 25x4.6 mm, Gradient: 100% A/0% B → 0% A/100% B während 60 Minuten, mit A=Wasser 0.1% TFA, B=Acetonitril 0.1% TFA, Retentionszeit 52 Min. Präparative HPLC: 10 mg werden in 500 µl Trifluorethanol-Acetonitril (1:1) gelöst, mit obigem Gradienten und Säule getrennt und die Hauptfraktion gesammelt und eingedampft. Auf diese Weise werden 6.8 mg eines Produkts erhalten, das gemäss HPLC mehr als 90% der Titelverbindung enthält.

### Beispiel 12: O-[Fmoc-Asp(OBut)-Arg(Mtr)-Gly-Phe-Tyr(But)-Phe-Ser(But)-Arg(Mtr)-Pro-Ala-Ser(But)-Arg(Mtr)-Val-Ser(But)-Arg(Mtr)-Arg(Mtr)-Ser(But)-Arg(Mtr)-Gly]-Hydroxylharz (Seitenkettengeschütztes IGF-2 (23-41)-nonadecapeptid an Hydroxylharz gebunden), (9)

1.20 g *(6)* (0.31 mMol) von Beispiel 8 werden in einem Peptidsynthesizer mit Waschprozessen und Fmoc-Abspaltprozessen analog Beispiel 6 umgesetzt. Alle Aminosäuren werden zur Kupplung (1 Stunde) als symmetrische Anydride (3 Aeq., Herstellung wie in Beispiel 10) eingesetzt. Mit den Aminosäuren 14, 17 und 18 (d.h. 36, 39 und 40 von IGF-2) wird der Kupplungsprozess wiederholt (1 Stunde). Nach Abschluss der Synthese wird das Harz 3x mit je 5 ml Isopropylakohol gewaschen und im Hochvakuum getrocknet.

### Beispiel 13: Fmoc-Asp(OBut)-Arg(Mtr)-Gly-Phe-Tyr(But)-Phe-Ser(But)-Arg(Mtr)-Pro-Ala-Ser(But)-Arg(Mtr)-Val-Ser(But)-Arg(Mtr)-Arg(Mtr)-Ser(But)-Arg(Mtr)-Gly-OH [Fmoc-geschütztes IGF-2(23-41)-nonadecapeptid]

1.65 g *(9)* (ca. 154 µMol) von Beispiel 12 werden mit 33 ml Dichlormethan-Essigsäure (9:1 Vol.) analog Beispiel 11 gespalten und gewaschen. Nach der Lyophilisation wird ein farbloses Pulver erhalten. Zur Reinigung wird dieses Produkt in einem automatischen Gegenstromverteilungsapparat (5 ml/Phase) mit dem Gemisch Methanol-Wasser-Chloroform-Tetrachlorkohlenstoff (2700:675:900:1575) über 1050 Stufen verteilt. Die Fraktionen 46-75 enthalten die reine Titelverbindung (DC, HPLC) und werden zusammen eingedampft, der Rückstand mit Ether verrieben und abfiltriert. Das so erhaltene farblose Pulver ist einheitlich nach DC (4 Systeme auf Kieselgel) und HPLC (System wie in Beispiel 11; Retentionszeit 57 Min.). FAB-MS (fast atom bombardment-Massenspektrum): korrekter Massenpeak (2480).

### Beispiel 14: Fmoc-Aminoharz (10) und Aminoharz [4-(2,4-Dimethoxyphenyl-amino-methyl)-phenoxymethylpolystyrol]

4.1 g Hydroxylharz *(3)* (ca. 1.89 mMol) werden mit 80 ml Dioxan angeschlämmt. 1.35 g 9-Fluorenylmethyl-carbamat (5.65 mMol) (hergestellt nach Carpino, L.A. et al., J.Org.Chem. 48, 661 (1983)) und 0.47 ml 1 M Benzolsulfonsäure in 1,2-Ethandichlorid (EDC) (0.47 mMol) werden zugefügt und die Mischung während 3 Stunden bei 50°C gehalten. Nach der Zugabe von weiteren 0.47 ml 1 M Benzolsulfonsäure wird noch 17 Stunden bei 50°C reagiert. Das Harz wird filtriert und mit 30 ml Portionen der folgenden Lösungsmittel gewaschen: 3x Isopropylalkohol, 3x EDC, 1x Dimethylacetamid (DMA), 6x Isopropylalkohol, 3x DMA. Das Harz wird dann zur Blockierung nicht umgesetzter Hydroxylgruppen mit 5.0 g Benzoesäureanhydrid in 25 ml DMA und 5 ml Pyridin während 2 Stunden bei Raumtemperatur gehalten und wie oben gewaschen. Die letzte Waschung erfolgt mit 6x Isopropylalkohol. Das so erhältliche Fmoc-Aminoharz *(10)* wird dann im Hochvakuum bis zur Massenkonstanz getrocknet. Die spektrophotometrische Bestimmung von Fmoc nach Spaltung einer Probe analog Beispiel 5 ergibt eine spezifische Beladung von ca. 0.35 mMol Fmoc/g Harz.

Zur Herstellung des freien Aminoharzes werden 2.0 g Fmoc-Aminoharz *(10)* in 30 ml 20% Piperidin in Dimethylacetamid (DMA) 1 Stunde bei Raumtemperatur gerührt, filtriert, mit weiteren 30 ml 20% Piperidin in DMa 1 Stunde nachbehandelt und wie folgt mit 30 ml-Portionen gewaschen: 3x DMA, 3x Isopropylalkohol, 3x DMA, 6x Isopropylalkohol. Das Harz wird im Hochvakuum bis zur Massenkonstanz getrocknet.

### Beispiel 15: N-[Met-His(Trt)-Glu(OBut)-Gly-Asp(OBut)-Glu(OBut)-Gly-Pro-Gly]-Aminoharz, (11)

0.50 g Fmoc-Aminoharz *(10)* (ca. 175 µMol) von Beispiel 14 werden in einem automatischen Peptidsynthesizer mittels des folgenden Prozesses durch nacheinander folgendes wechselweises Abspalten der Fmoc-Gruppe und Ankondensieren von Fmoc-Gly, Fmoc-Pro, Fmoc-Gly, Fmoc-Glu(OBut), Fmoc-Asp(OBut), Fmoc-Gly, Fmoc-Glu(OBut), Fmoc-His(Trt) und Fmoc-Met mit jeweiligem Blockieren von nicht umgesetzten Aminogruppen durch Acetylierung umgesetzt:
Waschen und Abspalten (Deblockieren) von Fmoc bei Raumtemperatur mit je ca. 10 ml: 1x Isopropylalkohol (1 Min.), 4x DMA entgast (0.5 Min.), 6x 20% Piperidin in DMA (2 Min.), 2x DMA entgast (0.5 Min.), 1x Isopropylalkohol (1 Min.), 4x DMA entgast (0.5 Min.), 3x DMA dest. (0.5 Min.).
Kupplung: 0.52 mMol geschützter Aminosäure-2,4,5-trichlorphenylester und 0.92 ml 0.57 M 1-Hydroxybenzotriazol (HOBt)/0.57 M Diisopropylethylamin in DMA (0.52 mMol) unter Zusatz von 0.88 ml DMA (30 Min. bei Raumtemperatur).
Waschen und Acetylieren der restlichen freien Aminogruppen mit: 1x Acetanhydrid-Pyridin-DMA (1:1:8 Vol.) (5 Min.), 3x DMA entgast (0.5 Min.), 1x Isopropylalkohol (1 Min.), 3x DMA entgast (0.5 Min.). Die endständige Fmoc-Gruppe wird wie oben beschrieben abgespalten. Das Harz wird mit Isopropylalkohol gewaschen und im Hochvakuum bei Raumtemperatur getrocknet.

### Beispiel 16: Met-His-Glu-Gly-Asp-Glu-Gly-Pro-Gly-NH₂ (MIF-related protein 14 (94-102)-amid)

0.65 g *(11)* (ca. 150 mMol) von Beispiel 15 werden in einer Säule während 60 Min. mit ca. 30 ml 2% Trifluoressigsäure (TFA) in Dichlormethan gewaschen, das Eluat im Vakuum auf ca. 0.3 ml konzentriert, 1 ml TFA-Wasser (95:5 Vol.) zugefügt und zur vollständigen Abspaltung der Schutzgruppen während 15 Min. bei Raumtemperatur belassen. Das Peptidamid wird durch Zugabe von 5 ml Diisopropylether ausgefällt. Die Fällung wird abfiltriert und im Vakuum getrocknet. Das Produkt wird in 5 ml Wasser gelöst, eine Trübung abzentrifugiert und der Ueberstand lyophilisiert. Erhalten wird ein farbloses Pulver, welches gemäss HPLC die Titelverbindung zu über 90% enthält. HPLC: Retentionszeit 6.8 Min., Säule Nucleosil 7C₁₈, 120x4.6 mm, Gradient: 100% A/0% B → 10% A/90% B in 30 Min. mit A=0,1% TFA/Wasser, B=0.1% TFA/Acetonitril, 1.5 ml/Min., Detektion bei 215 nm. FAB-MS: Massenpeak 927 (M+H⁺).

### Beispiel 17: O-[Fmoc-Ala-Tyr(But)-Arg(Mtr)-Pro-Ser(But)-Glu(OBut)-Thr(But)-Leu-Cys(Trt)-Gly-Gly-Glu(OBut)-Leu-Val-Asp(OBut)-Thr(But)-Leu-Gln-Phe-Val-Cys(SBut)-Gly]-Hydroxylharz (Seitenkettengeschütztes IGF-2 (1-22)-dokosapeptid an Hydroxylharz gebunden), (12)

1.00 g O-[Fmoc-Gly]-Hydroxylharz *(6)* (ca. 0.36 mMol) werden in einem Peptidsynthesizer mit Waschprozessen und Fmoc-Abspaltprozessen analog Beispiel 15 umgesetzt.
Kupplung: Val, Gln, Gly, Leu und Arg werden als Fmoc-aminosäure-2,4,5-trichlorphenylester (0.72 mMol) in 1.20 ml DMA zusammen mit 1.44 ml 0.5 M HOBt/0.5 M Diisopropylethylamin in DMA bei Raumtemperatur während 1 Stunde gekuppelt. Die anderen Aminosäurederivate werden als symmetrische Anhydride (1.08 mMol) (Darstellung analog Beispiel 10) während 1 Stunde bei Raumtemperatur angekuppelt. Bei Aminosäure Nr. 3, 8, 10, 11, 18, 20 und 21 wird der Prozess wiederholt (Nachkupplung).
Nach Abschluss der Synthese wird das Harz unter Belassung der endständigen Fmoc-Gruppe 5x mit Isopropylalkohol gewaschen und im Hochvakuum getrocknet.

### Beispiel 18: Fmoc-Ala-Tyr(But)-Arg(Mtr)-Pro-Ser(But)-Glu(OBut)-Thr(But)-Leu-Cys(Trt)-Gly-Gly-Glu(OBut)-Leu-Val-Asp(OBut)-Thr(But)-Leu-Gln-Phe-Val-Cys(SBut)-Gly-OH (Seitenkettengeschütztes IGF-2 (1-22)-dokosapeptid)

1.80 g *(12)* (ca. 0.19 mMol) von Beispiel 17 werden mit 35 ml Dichlormethan-Essigsäure (9:1 Vol.) während 1.5 Stunden bei Raumtemperatur gemischt. Das Harz wird abfiltriert und das schwerlösliche Fragment bei 60°C wie folgt extrahiert: 3x 50 ml Dimethylsulfoxid (DMSO), 3x Trifluorethanol-Dichlormethan (1:1 Vol.), 1x N-Methylpyrrolidon-DMSO (8:2 Vol.). Die kombinierten Filtrate werden im Hochvakuum eingedampft und der Rückstand mit 5 ml Wasser-Methanol (5:95 Vol.) bei 50°C während 1 Stunde verrührt. Das ungelöste Material wird abfiltriert und im Hochvakuum bei Raumtemperatur getrocknet. Das extrem schwerlösliche Fragment kann mittels HPLC-Technik nicht analysiert werden. DC (2 Systeme): Das Produkt ist mehr als 90-prozentig (gelöst in DMSO-N-Methylpyrrolidon (2:8)). FAB-MS: Massenpeak 3539 (M+Na⁺).

### Beispiel 19: O-[Fmoc-Ala-Tyr(But)-Arg(Mtr)-Pro-Ser(But)-Glu(OBut)-Thr(But)-Leu-Cys(Trt)-Gly]-Hydroxylharz (Seitenkettengeschütztes IGF-2 (1-10)-undekapeptid an Hydroxylharz gebunden), (13)

1.00 g O-[Fmoc-Gly]-Hydroxylharz *(6)* (ca. 0.36 mMol) werden in einem Peptidsynthesizer mit Waschprozessen und Fmoc-Abspaltprozessen analog Beispiel 15 umgesetzt.
Kupplung: Die Aminosäurederivate werden als symmetrische Anhydride (1.08 mMol) (Darstellung analog Beispiel 10) während 1 Stunde bei Raumtemperatur angekuppelt. Bei Aminosäure Nr. 3, 8 und 9 wird der Kupplungsprozess mit 2 Aequivalenten 2,4,5-Trichlorphenylester wiederholt. Nach Abschluss der Synthese wird das Harz unter Belassung der endständigen Fmoc-Gruppe 5x mit Isopropylalkohol gewaschen und im Hochvakuum getrocknet.

### Beispiel 20: Fmoc-Ala-Tyr(But)-Arg(Mtr)-Pro-Ser(But)-Glu(OBut)-Thr(But)-Leu-Cys(Trt)-Gly (Seitenkettengeschütztes IGF-2 (1-10)-undekapeptid)

1.50 g *(13)* (ca. 0.19 mMol) von Beispiel 19 werden mit 30 ml Dichlormethan-Essigsäure (9:1 Vol.) während 1.5 Stunden bei Raumtemperatur gemischt. Das Harz wird abfiltriert, das Filtrat im Vakuum konzentriert und im Hochvakuum lyophilisiert. Das so erhaltene farblose Pulver wird zur Reinigung in einem automatischen Gegenstromverteilungsapparat (5 ml/Phase) mit dem gleichen Gemisch wie in Beispiel 13 über 1330 Stufen verteilt. Die Fraktionen 65-111 enthalten die reine Titelverbindung (DC, HPLC) und werden zusammen eingedampft. Der Rückstand wird mit Ether verrieben und abfiltriert. Das farblose Pulver ist einheitlich nach DC (5 Systeme auf Kieselgel) und HPLC. Retentionszeit 24.1 Min., Säule Nucleosil 5C₁₈, 4.6x250 mm, Gradient 50% A/50% B → 0% A/100% B in 30 Min. mit A=0.1 % TFA/Wasser, B=0.1 % TFA/Acetonitril, 1.0 ml/Min., Detektion bei 215 nm.

### Beispiel 21: O-[Asn-Phe-Phe-D-Trp-Lys(Boc)-Thr(But)-Phe-Gaba]-Hydroxylharz (Seitenkettengeschütztes Somatostatin-D-Trp(8)-4-aminobuttersäure(12)-(5-12)-octapeptid an Hydroxylharz gebunden), (14)

1.00 g O-[Fmoc-Gaba]-Hydroxylharz (hergestellt analog Beispiel 8 mit 4-Aminobuttersäure statt Glycin) (ca. 0.31 mMol) wird in einem Peptidsynthesizer mit Waschprozessen und Fmoc-Abspaltprozessen analog Beispiel 15 umgesetzt.
Kupplung: Die Aminosäurederivate werden als symmetrische Anhydride (0.93 mMol) (Darstellung analog Beispiel 10) während 1 Stunde bei Raumtemperatur angekuppelt. Phe und Asn werden als 2,4,5-Trichlorphenylester (2 Aequivalente) gekuppelt. Mit Aminosäure 1 (Asn) wird eine Nachkupplung durchgeführt. Nach Abschluss der Synthese und nach Abspaltung der endständigen Fmoc-Gruppen wird das Harz mit 5x Isopropylalkohol gewaschen und im Hochvakuum getrocknet.

### Beispiel 22: H-Asn-Phe-Phe-D-Trp-Lys(Boc)-Thr(But)-Phe-Gaba-OH (Seitenkettengeschütztes Somatostatin-D-Trp(8)-4-aminobuttersäure(12)-(5-12)-octapeptid)

1.38 g *(14)* (ca. 0.26 mMol) von Beispiel 21 werden mit 5 ml 5% Pyridinhydrochlorid in DMA während 1 Stunde bei 50°C gemischt. Das Harz wird abfiltriert und mit DMSO gewaschen, das Filtrat im Hochvakuum konzentriert und 1x aus DMSO und 2x aus tert-Butylalkohol lyophilisiert. Das so erhaltene farblose Pulver ist gemäss HPLC zu über 95 % rein und ist im HPLC und DC identisch mit einer authentischen Probe, welche durch Synthese in Lösung hergestellt worden ist. HPLC: Retentionszeit 20.6 Min., Säule Nucleosil 5C₁₈, 4.6x250 mm, Gradient 100% A/0% B → 10% A/90% B in 30 Min., mit A=0.1 % TFA/Wasser, B=0.1 % TFA/Acetonitril, 1.0 ml/Min., Detektion bei 215 nm.

## Patentansprüche (Patentansprüche für folgende(n) Vertragsstaat(en): AT, BE, CH, DE, FR, GB, GR, IT, LI, LU, NL, SE)

1. Ein Kunstharz auf der Basis eines als Träger für Festphase-Peptidsynthese verwendbaren, mit 0-5 Mol% Divinylbenzol quervernetzten Polystyrols, dadurch gekennzeichnet, dass es an Benzolringen seines Grundgerüsts durch die Gruppen der Formel 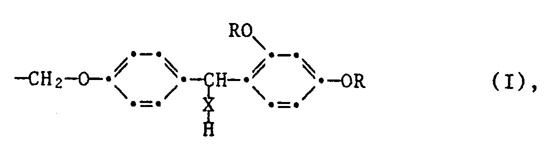 worin X für -O- oder -NH- und R für C₁₋₄-Alkyl steht, substituiert ist.

2. Ein Kunstharz gemäss Anspruch 1, worin das Polystyrol als Copolymer mit 1-2% Divinylbenzol vorliegt.

3. Ein Kunstharz gemäss Anspruch 1 oder 2, worin in Formel I X für -O- und R für Methyl steht.

4. Ein Kunstharz gemäss Anspruch 1 oder 2, worin in Formel I X für -NH- und R für Methyl steht.

5. Verfahren zur Herstellung eines Kunstharzes auf der Basis eines als Träger für Festphase-Peptidsynthese verwendbaren, mit 0-5 Mol% Divinylbenzol quervernetzten Polystyrols, welches an Benzolringen seines Grundgerüsts durch die Gruppen der Formel 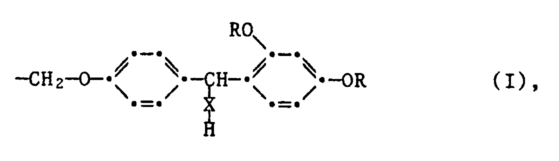 worin X für -O- oder -NH- und R für C₁₋₄-Alkyl steht, substituiert ist, dadurch gekennzeichnet, dass man ein als Träger für Festphase-Peptidsynthese anwendbares, mit 0-5% Divinylbenzol quervernetztes und an Benzolringen des Grundgerüsts chlormethyliertes oder brommethyliertes Polystyrol nacheinander
a) mit einer Verbindung der Formel 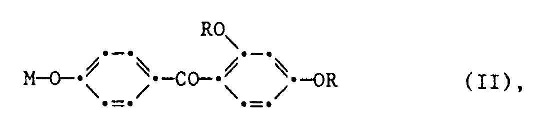 worin M ein Alkalimetall ist und R die obgenannte Bedeutung hat,
b) mit einem Reduktionsmittel, und, falls X für -NH- steht,
c) mit einem die Aminogruppe einführenden Reagens umsetzt.

6. Verfahren gemäss Anspruch 5, dadurch gekennzeichnet, dass man mit einer Verbindung der Formel II umsetzt, worin M für Cäsium steht.

7. Verfahren gemäss Anspruch 5 oder 6, dadurch gekennzeichnet, dass man in Schritt b) mit einem komplexen Hydrid oder Diboran reduziert.

8. Verfahren gemäss einem der Ansprüche 5 bis 7, dadurch gekennzeichnet, dass man mit einer Verbindung der Formel II umsetzt, worin R für Methyl steht.

9. Verfahren zur Herstellung von Peptiden und Peptidamiden, dadurch gekennzeichnet, dass man ein in Anspruch 1 definiertes Kunstharz als Träger für die Festphase-Synthese verwendet.

10. Verfahren gemäss Anspruch 9, dadurch gekennzeichnet, dass man am N-Terminus und/oder an sonstigen funktionellen Gruppen geschützte Peptide oder Peptidamide herstellt.

11. Verfahren gemäss Anspruch 9 oder 10, dadurch gekennzeichnet, dass man
a) das Kunstharz mit einer Verbindung der Formel W¹-AM¹-Y-H, worin Y für -O- oder -NH-, W¹ für eine N-terminale Aminoschutzgruppe und AM¹ für den Acylrest einer an funktionellen Gruppen gewünschtenfalls geschützten Aminosäuresequenz bestehend aus 1-25 Aminosäureresten steht, oder mit einem reaktionsfähigen funktionellen Derivat davon zwecks Verankerung des Rests W¹-AM¹- an der Gruppe -X- des Harzes umsetzt,
b) die N-terminale Schutzgruppe W¹ abspaltet,
c) die freigewordene N-terminale Aminogruppe durch Umsetzen mit einer Säure der Formel W²-AM²-OH, worin W² und AM² analoge Bedeutung wie die oben definierten Reste W¹ und AM¹ haben, oder mit einem reaktionsfähigen funktionellen Derivat davon acyliert,
d) den Vorgang der wechselweisen Abspaltung gemäss b) und Acylierung gemäss c) beliebige Male bis zum Erreichen einer gewünschten Aminosäuresequenz wiederholt und
e) das gebildete Peptid oder Peptidamid, gewünschtenfalls nach vorangehender oder mit gleichzeitiger Abspaltung von Schutzgruppen, durch Acidolyse vom Harz ablöst.

12. Verfahren gemäss Anspruch 11, dadurch gekennzeichnet, dass man zum Schutz der N-terminalen Aminogruppe eine basisch abspaltbare Aminoschutzgruppe W¹ bzw. W² verwendet und die Abspaltung gemäss Verfahrensstufe b) mit einer Base vornimmt.

13. Verfahren gemäss Anspruch 12, dadurch gekennzeichnet, dass man als basisch abspaltbare Aminoschutzgruppe W¹ bzw. W² die 9-Fluorenylmethoxycarbonylgruppe (Fmoc) verwendet.

14. Verfahren gemäss einem der Ansprüche 11 bis 13, dadurch gekennzeichnet, dass man in Verfahrensstufe a) ein Kunstharz, in welchem X für -O- steht, mit einer Säure der Formel W¹-AM¹-OH, worin W¹ und AM¹ die im Anspruch 11 angegebenen Bedeutungen haben, oder mit einem reaktiven funktionellen Derivat davon umsetzt und das Endprodukt in Säureform abspaltet.

15. Verfahren gemäss einem der Ansprüche 11 bis 13, dadurch gekennzeichnet, dass man in Verfahrensstufe a) ein Kunstharz, in welchem X für -O- steht, mit einem Amid der Formel W¹-AM¹-NH₂, worin W¹ und AM¹ die im Anspruch 11 angegebenen Bedeutungen haben, umsetzt und das Endprodukt in Form eines Peptidamids abspaltet.

16. Verahren gemäss einem der Ansprüche 11 bis 13, dadurch gekennzeichnet, dass man in Verfahrensstufe a) ein Kunstharz, in welchem X für -NH- steht, mit einer Säure der Formel W¹-AM¹-OH, worin W¹ und AM¹ die im Anspruch 11 angegebenen Bedeutungen haben, oder mit einem reaktiven funktionellen Derivat davon umsetzt und das Endprodukt in Form eines Peptidamids abspaltet.

17. Verwendung eines Kunstharzes gemäss Anspruch 1 als Träger bei der Festphase-Synthese von Peptiden und Peptidamiden.

18. Ein Kunstharz der im Anspruch 1 definierten Struktur, welches anstelle der Gruppe -X-H die Gruppe -NH-W, -X-AM-W oder -X-AM-H trägt, worin -X- für -O- oder -NH-, W für eine N-terminale Aminoschutzgruppe und AM für den Acylrest einer an funktionellen Gruppen gewünschtenfalls geschützten Aminosäuresequenz bestehend aus 1-180 Aminosäurereste steht.

19. Ein durch das Verfahren gemäss Anspruch 11 erhaltenes Peptid oder Peptidamid der Formel W-AM⁰-XH oder der Formel H-AM⁰-XH, worin -X- für -O- oder -NH-, W für eine N-terminale Aminoschutzgruppe und AM⁰ für den Acylrest einer an funktionellen Gruppen gewünschtenfalls geschützten Aminosäuresequenz bestehend aus 2-180 Aminosäurereste steht.

## Patentansprüche (Patentansprüche für folgende(n) Vertragsstaat(en): ES)

1. Verfahren zur Herstellung eines Kunstharzes auf der Basis eines als Träger für Festphase-Peptidsynthese verwendbaren, mit 0-5 Mol% Divinylbenzol quervernetzten Polystyrols, welches an Benzolringen seines Grundgerüsts durch die Gruppen der Formel 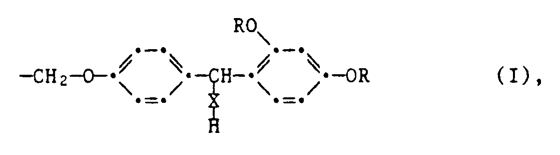 worin X für -O- oder -NH- und R für C₁₋₄-Alkyl steht, substituiert ist, dadurch gekennzeichnet, dass man ein als Träger für Festphase-Peptidsynthese anwendbares, mit 0-5% Divinylbenzol quervernetztes und an Benzolringen des Grundgerüsts chlormethyliertes oder brommethyliertes Polystyrol nacheinander
a) mit einer Verbindung der Formel 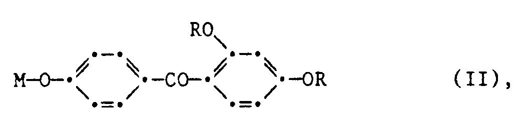 worin M ein Alkalimetall ist und R die obgenannte Bedeutung hat,
b) mit einem Reduktionsmittel, und, falls X für -NH- steht,
c) mit einem die Aminogruppe einführenden Reagens umsetzt.

2. Verfahren gemäss Anspruch 1, dadurch gekennzeichnet, dass man mit 1-2 % Divinylbenzol quervernetztes Polystyrol einsetzt.

3. Verfahren gemäss Anspruch 1 oder 2, dadurch gekennzeichnet, dass man mit einer Verbindung der Formel II umsetzt, worin M für Cäsium steht.

4. Verfahren gemäss einem der Ansprüche 1 bis 3, dadurch gekennzeichnet, dass man in Schritt b) mit einem komplexen Hydrid oder Diboran reduziert.

5. Verfahren gemäss einem der Ansprüche 1 bis 4, dadurch gekennzeichnet, dass man mit einer Verbindung der Formel II umsetzt, worin R für Methyl steht.

6. Verfahren gemäss Anspruch 5 zur Herstellung eines Kunstharzes, worin in Formel I X für -O- und R für Methyl steht.

7. Verfahren gemäss Anspruch 5 zur Herstellung eines Kunstharzes, worin in Formel I X für -NH- und R für Methyl steht.

8. Verfahren zur Herstellung von Peptiden und Peptidamiden, dadurch gekennzeichnet, dass man ein in Anspruch 1 definiertes Kunstharz als Träger für die Festphase-Synthese verwendet.

9. Verfahren gemäss Anspruch 8, dadurch gekennzeichnet, dass man am N-Terminus und/oder an sonstigen funktionellen Gruppen geschützte Peptide oder Peptidamide herstellt.

10. Verfahren gemäss Anspruch 8 oder 9, dadurch gekennzeichnet, dass man
a) das Kunstharz mit einer Verbindung der Formel W¹-AM¹-Y-H, worin Y für -O- oder -NH-, W¹ für eine N-terminale Aminoschutzgruppe und AM¹ für den Acylrest einer an funktionellen Gruppen gewünschtenfalls geschützten Aminosäuresequenz bestehend aus 1-25 Aminosäureresten steht, oder mit einem reaktionsfähigen funktionellen Derivat davon zwecks Verankerung des Rests W¹-AM¹- an der Gruppe -X- des Harzes umsetzt,
b) die N-terminale Schutzgruppe W¹ abspaltet,
c) die freigewordene N-terminale Aminogruppe durch Umsetzen mit einer Säure der Formel W²-AM²-OH, worin W² und AM² analoge Bedeutung wie die oben definierten Reste W¹ und AM¹ haben, oder mit einem reaktionsfähigen funktionellen Derivat davon acyliert,
d) den Vorgang der wechselweisen Abspaltung gemäss b) und Acylierung gemäss c) beliebige Male bis zum Erreichen einer gewünschten Aminosäuresequenz wiederholt und
e) das gebildete Peptid oder Peptidamid, gewünschtenfalls nach vorangehender oder mit gleichzeitiger Abspaltung von Schutzgruppen, durch Acidolyse vom Harz ablöst.

11. Verfahren gemäss Anspruch 10, dadurch gekennzeichnet, dass man zum Schutz der N-terminalen Aminogruppe eine basisch abspaltbare Aminoschutzgruppe W¹ bzw. W² verwendet und die Abspaltung gemäss Verfahrensstufe b) mit einer Base vornimmt.

12. Verfahren gemäss Anspruch 11, dadurch gekennzeichnet, dass man als basisch abspaltbare Aminoschutzgruppe W¹ bzw. W² die 9-Fluorenylmethoxycarbonylgruppe (Fmoc) verwendet.

13. Verfahren gemäss einem der Ansprüche 10 bis 12, dadurch gekennzeichnet, dass man in Verfahrensstufe a) ein Kunstharz, in welchem X für -O- steht, mit einer Säure der Formel W¹-AM¹-OH, worin W¹ und AM¹ die im Anspruch 11 angegebenen Bedeutungen haben, oder mit einem reaktiven funktionellen Derivat davon umsetzt und das Endprodukt in Säureform abspaltet.

14. Verfahren gemäss einem der Ansprüche 10 bis 12, dadurch gekennzeichnet, dass man in Verfahrensstufe a) ein Kunstharz, in welchem X für -O- steht, mit einem Amid der Formel W¹-AM¹-NH₂, worin W¹ und AM¹ die im Anspruch 11 angegebenen Bedeutungen haben, umsetzt und das Endprodukt in Form eines Peptidamids abspaltet.

15. Verfahren gemäss einem der Ansprüche 10 bis 12, dadurch gekennzeichnet, dass man in Verfahrensstufe a) ein Kunstharz, in welchem X für -NH- steht, mit einer Säure der Formel W¹-AM¹-OH, worin W¹ und AM¹ die im Anspruch 11 angegebenen Bedeutungen haben, oder mit einem reaktiven funktionellen Derivat davon umsetzt und das Endprodukt in Form eines Peptidamids abspaltet.

16. Verfahren gemäss Anspruch 8 oder 9, dadurch gekennzeichnet, dass die Peptide oder Peptidamide aus 2-180 Aminosäureresten bestehen.

17. Verfahren zur Herstellung eines Kunstharzes der im Anspruch 1 definierten Struktur, welches anstelle der Gruppe -X-H die Gruppe -X-AM-W oder -X-AM-H trägt, worin -X- für -O- oder -NH-, W für eine N-terminale Aminoschutzgruppe und AM für den Acylrest einer an funktionellen Gruppen gewünschtenfalls geschützten Aminosäuresequenz bestehend aus 1-180 Aminosäurereste steht, dadurch gekennzeichnet, dass man ein im Anspruch 1 definiertes Kunstharz
a) mit einer Verbindung der Formel W¹-AM¹-Y-H, worin Y für -O- oder -NH-, W¹ für eine N-terminale Aminoschutzgruppe und AM¹ für den Acylrest einer an funktionellen Gruppen gewünschtenfalls geschützten Aminosäuresequenz bestehend aus 1-25 Aminosäureresten steht, oder mit einem reaktionsfähigen funktionellen Derivat davon zwecks Verankerung des Rests W¹-AM¹- an der Gruppe -X- des Harzes umsetzt, gegebenenfalls
b) die N-terminale Schutzgruppe W¹ abspaltet, gegebenenfalls
c) die freigewordene N-terminale Aminogruppe durch Umsetzen mit einer Säure der Formel W²-AM²-OH, worin W² und AM² analoge Bedeutung wie die oben definierten Reste W¹ und AM¹ haben, oder mit einem reaktionsfähigen funktionellen Derivat davon acyliert, und
d) den Vorgang der wechselweisen Abspaltung gemäss b) und Acylierung gemäss c) beliebige Male bis zum Erreichen einer gewünschten Aminosäuresequenz wiederholt.

18. Verfahren zur Herstellung eines Kunstharzes der im Anspruch 1 definierten Struktur, welches anstelle der Gruppe -X-H die Gruppe -NH-W trägt, worin W für eine N-terminale Aminoschutzgruppe steht, dadurch gekennzeichnet, dass man ein im Anspruch 1 definiertes Kunstharz mit einer Verbindung der Formel W-NH₂, worin W die genannte Bedeutung hat, oder mit einem reaktionsfähigen funktionellen Derivat davon zwecks Verankerung des Restes W-NH- an der Gruppe -X- des Harzes umsetzt.

## Claims (Claims for the following Contracting State(s): AT, BE, CH, DE, FR, GB, GR, IT, LI, LU, NL, SE)

1. A synthetic resin based on a polystyrene that can be used as a support for solid phase peptide synthesis and that has been cross-linked with from 0 to 5 mol % of divinyl benzene, characterised in that it has been substituted at benzene rings of its skeletal structure by groups of the formula 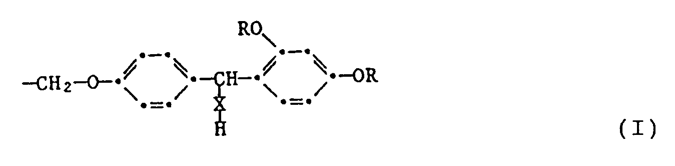 in which X represents -O- or -NH- and R represents C₁-C₄-alkyl.

2. A synthetic resin according to claim 1 in which the polystyrene is in the form of a copolymer with from 1 to 2 % divinyl benzene.

3. A synthetic resin according to claim 1 or 2 in which in formula I X represents -O- and R represents methyl.

4. A synthetic resin according to claim 1 or 2 in which in formula I X represents -NH- and R represents methyl.

5. A process for the manufacture of a synthetic resin based on a polystyrene that can be used as a support for solid phase peptide synthesis, that has been cross-linked with from 0 to 5 mol % of divinyl benzene, and that has been substituted at benzene rings of its skeletal structure by groups of the formula 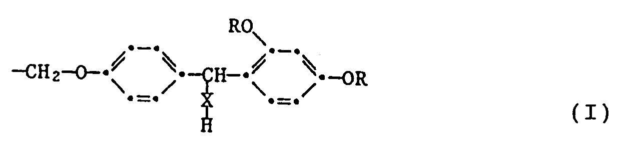 in which X represents -O- or -NH- and R represents C₁-C₄-alkyl, characterised in that a polystyrene that can be used as a support for solid phase peptide synthesis, has been cross-linked with from 0 to 5 % of divinyl benzene and has been chloromethylated or bromomethylated at benzene rings of its skeletal structure is reacted in succession
a) with a compound of formula 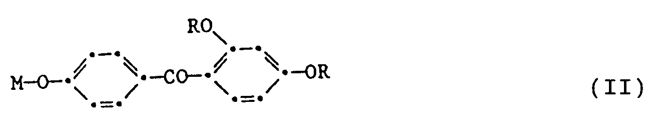 in which M is an alkali metal and R has the meaning mentioned hereinbefore,
b) with a reducing agent and, if X represents -NH-,
c) with a reagent that introduces the amino group.

6. A process according to claim 5, characterised in that the reaction is carried out with a compound of formula II in which M represents caesium.

7. A process according to claim 5 or 6, characterised in that in step b) reduction is carried out with a complex hydride or diborane.

8. A process according to any one of claims 5 to 7, characterised in that the reaction is carried out with a compound of formula II in which R represents methyl.

9. A process for the manufacture of peptides and peptide amides, characterised in that a synthetic resin defined in claim 1 is used as a support for the solid phase synthesis.

10. A process according to claim 9, characterised in that peptides or peptide amides protected at the N-terminal and/or at other functional groups are manufactured.

11. A process according to claim 9 or 10, characterised in that
a) the synthetic resin is reacted with a compound of formula W¹-AM¹-Y-H in which Y represents -O- or -NH-, W¹ represents an N-terminal amino-protecting group and AM¹ represents the acyl radical of an amino acid sequence consisting of from 1 to 25 amino acid residues which is, if desired, protected at functional groups, or with a reactive functional derivative thereof, for the purpose of attaching the W¹-AM¹- residue to the -X- group of the resin,
b) the N-terminal protecting group W¹ is removed,
c) the freed N-terminal amino group is acylated by reaction with an acid of formula W²-AM²-OH in which W² and AM² have meanings analogous to those of the above-defined radicals W¹ and AM¹, or with a reactive functional derivative thereof,
d) the operation of alternate removal according to b) and acylation according to c) is repeated as required until the desired amino acid sequence is obtained and
e) the resulting peptide or peptide amide, if desired after removal or with simultaneous removal of protecting groups, is detached from the resin by acidolysis.

12. A process according to claim 11, characterised in that an amino-protecting group W¹ or W² that can be removed under basic conditions is used to protect the N-terminal amino group and the removal according to process step b) is carried out with a base.

13. A process according to claim 12, characterised in that the 9-fluorenylmethoxycarbonyl group (Fmoc) is used as the amino-protecting group W¹ or W² that can be removed under basic conditions.

14. A process according to any one of claims 11 to 13, characterised in that in process step a) a synthetic resin in which X represents -O- is reacted with an acid of formula W¹-AM¹-OH in which W¹ and AM¹ have the meanings given in claim 11, or with a reactive functional derivative thereof, and the end product is removed in acidic form.

15. A process according to any one of claims 11 to 13, characterised in that in process step a) a synthetic resin in which X represents -O- is reacted with an amide of formula W¹-AM¹-NH₂ in which W¹ and AM¹ have the meanings given in claim 11, and the end product is removed in the form of a peptide amide.

16. A process according to any one of claims 11 to 13, characterised in that in process step a) a synthetic resin in which X represents -NH- is reacted with an acid of formula W¹-AM¹-OH in which W¹ and AM¹ have the meanings given in claim 11, or with a reactive functional derivative thereof, and the end product is removed in the form of a peptide amide.

17. The use of a synthetic resin according to claim 1 as a support in the solid phase synthesis of peptides and peptide amides.

18. A synthetic resin of the structure defined in claim 1, that instead of the -X-H group carries a -NH-W, -X-AM-W or X-AM-H group in which -X- represents -O- or -NH-, W represents an N-terminal amino-protecting group and AM represents the acyl radical of an amino acid sequence consisting of from 1 to 180 amino acid residues which is, if desired, protected at functional groups.

19. A peptide or peptide amide obtained by the process according to claim 11 of formula W-AM⁰-XH or of formula H-AM⁰-XH in which -X- represents -O- or -NH-, W represents an N-terminal amino-protecting group and AM⁰ represents the acyl radical of an amino acid sequence consisting of from 2 to 180 amino acid residues which is, if desired, protected at functional groups.

## Claims (Claims for the following Contracting State(s): ES)

1. A process for the manufacture of a synthetic resin based on a polystyrene that can be used as a support for solid phase peptide synthesis, that has been cross-linked with from 0 to 5 mol % of divinyl benzene, and that has been substituted at benzene rings of its skeletal structure by groups of the formula 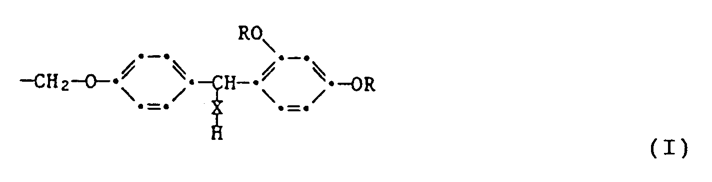 in which X represents -O- or -NH- and R represents C₁-C₄-alkyl, characterised in that a polystyrene that can be used as a support for solid phase peptide synthesis, has been cross-linked with from 0 to 5 % of divinyl benzene and has been chloromethylated or bromomethylated at benzene rings of its skeletal structure is reacted in succession
a) with a compound of formula 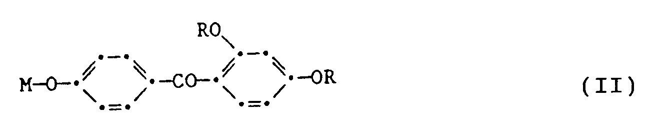 in which M is an alkali metal and R has the meaning mentioned hereinbefore,
b) with a reducing agent and, if X represents -NH-,
c) with a reagent that introduces the amino group.

2. A process according to claim 1, characterised in that polystyrene cross-linked with from 1 to 2 % divinyl benzene is used.

3. A process according to claim 1 or 2, characterised in that the reaction is carried out with a compound of formula II in which M represents caesium.

4. A process according to any one of claims 1 to 3, characterised in that in step b) reduction is carried out with a complex hydride or diborane.

5. A process according to any one of claims 1 to 4, characterised in that the reaction is carried out with a compound of formula II in which R represents methyl.

6. A process according to claim 5 for the manufacture of a synthetic resin in which in formula I X represents -O- and R represents methyl.

7. A process according to claim 5 for the manufacture of a synthetic resin in which in formula I X represents -NH- and R represents methyl.

8. A process for the manufacture of peptides and peptide amides, characterised in that a synthetic resin defined in claim 1 is used as a support for the solid phase synthesis.

9. A process according to claim 8, characterised in that peptides or peptide amides protected at the N-terminal and/or at other functional groups are manufactured.

10. A process according to claim 8 or 9, characterised in that
a) the synthetic resin is reacted with a compound of formula W¹-AM¹-Y-H in which Y represents -O- or -NH-, W¹ represents an N-terminal amino-protecting group and AM¹ represents the acyl radical of an amino acid sequence consisting of from 1 to 25 amino acid residues which is, if desired, protected at functional groups, or with a reactive functional derivative thereof, for the purpose of attaching the W¹-AM¹- residue to the -X- group of the resin,
b) the N-terminal protecting group W¹ is removed,
c) the freed N-terminal amino group is acylated by reaction with an acid of formula W²-AM²-OH in which W² and AM² have meanings analogous to those of the above-defined radicals W¹ and AM¹, or with a reactive functional derivative thereof,
d) the operation of alternate removal according to b) and acylation according to c) is repeated as required until the desired amino acid sequence is obtained and
e) the resulting peptide or peptide amide, if desired after removal or with simultaneous removal of protecting groups, is detached from the resin by acidolysis.

11. A process according to claim 10, characterised in that an amino-protecting group W¹ or W² that can be removed under basic conditions is used to protect the N-terminal amino group and the removal according to process step b) is carried out with a base.

12. A process according to claim 11, characterised in that the 9-fluorenylmethoxycarbonyl group (Fmoc) is used as the amino-protecting group W¹ or W² that can be removed under basic conditions.

13. A process according to any one of claims 10 to 12, characterised in that in process step a) a synthetic resin in which X represents -O- is reacted with an acid of formula W¹-AM¹-OH in which W¹ and AM¹ have the meanings given in claim 11, or with a reactive functional derivative thereof, and the end product is removed in acidic form.

14. A process according to any one of claims 10 to 12, characterised in that in process step a) a synthetic resin in which X represents -O- is reacted with an amide of formula W¹-AM¹-NH₂ in which W¹ and AM¹ have the meanings given in claim 11, and the end product is removed in the form of a peptide amide.

15. A process according to any one of claims 10 to 12, characterised in that in process step a) a synthetic resin in which X represents -NH- is reacted with an acid of formula W¹-AM¹-OH in which W¹ and AM¹ have the meanings given in claim 11, or with a reactive functional derivative thereof, and the end product is removed in the form of a peptide amide.

16. A process according to claim 8 or 9, characterised in that the peptides or peptide amides consist of from 2 to 180 amino acid residues.

17. A process for the manufacture of a synthetic resin of the structure defined in claim 1, that instead of the -X-H group carries a -X-AM-W or X-AM-H group in which -X- represents -O- or -NH-, W represents an N-terminal amino-protecting group and AM represents the acyl radical of an amino acid sequence consisting of from 1 to 180 amino acid residues which is, if desired, protected at functional groups, characterised in that a synthetic resin defined in claim 1
a) is reacted with a compound of formula W¹-AM¹-Y-H in which Y represents -O- or -NH-, W¹ represents an N-terminal amino-protecting group and AM¹ represents the acyl radical of an amino acid sequence consisting of from 1 to 25 amino acid residues which is, if desired, protected at functional groups, or with a reactive functional derivative thereof, for the purpose of attaching the W¹-AM¹- residue to the -X- group of the resin, if desired
b) the N-terminal protecting group W¹ is removed, if desired
c) the freed N-terminal amino group is acylated by reaction with an acid of formula W²-AM²-OH in which W² and AM² have meanings analogous to those of the above-defined radicals W¹ and AM¹, or with a reactive functional derivative thereof, and
d) the operation of alternate removal according to b) and acylation according to c) is repeated as required until the desired amino acid sequence is obtained.

18. A process for the manufacture of a synthetic resin of the structure defined in claim 1, that instead of the -X-H group carries a -NH-W group in which W represents an N-terminal amino-protecting group, characterised in that a synthetic resin defined in claim 1 is reacted with a compound of formula W-NH₂ wherein W has the meaning given, or with a reactive functional derivative thereof. for the purpose of attaching the W-NH- residue to the -X- group of the resin.

## Revendications (Revendications pour l'(les) Etat(s) contractant(s) suivant(s): AT, BE, CH, DE, FR, GB, GR, IT, LI, LU, NL, SE)

1. Une résine synthétique à base d'un polystyrène réticulé par 0 à 5 mol% de divinylbenzène et utilisable en tant que support pour des synthèses de peptides en phase solide, caractérisée en ce qu'elle est substituée sur les noyaux benzéniques de son squelette par les groupes de formule 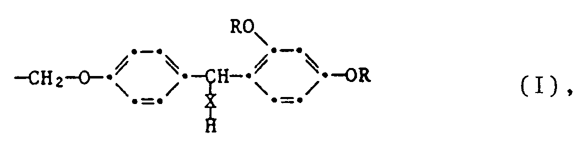 dans laquelle X représente -O- ou -NH- et R représente un groupe alkyle en C 1-C 4.

2. Une résine synthétique selon revendication 1, dans laquelle le polystyrène est à l'état de copolymère avec 1 à 2% de divinylbenzène.

3. Une résine synthétique selon revendication 1 ou 2, pour laquelle, dans la formule I, X représente -O- et R un groupe méthyle.

4. Une résine synthétique selon revendication 1 ou 2, pour laquelle, dans la formule I, X représente -NH- et R un groupe méthyle.

5. Procédé de préparation d'une résine synthétique à base d'un polystyrène réticulé par 0 à 5 mol% de divinylbenzène et utilisable en tant que support pour des synthèses de peptides en phase solide, qui est substituée sur les noyaux benzéniques de son squelette par les groupes de formule 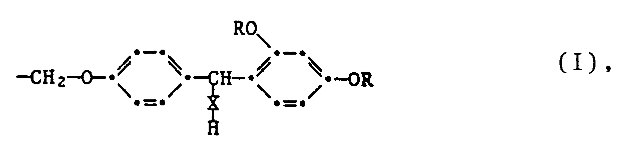 dans laquelle X représente -O- ou -NH- et R représente un groupe alkyle en C 1-C 4, caractérisé en ce que, partant d'un polystyrène réticulé par 0 à 5% de divinylbenzène et chlorométhylé ou bromométhylé sur les noyaux benzéniques du squelette, lui-même utilisable en tant que support pour la synthèse des peptides en phase solide, on le fait réagir successivement
a) avec un composé de formule 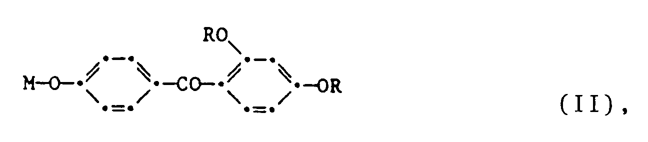 dans laquelle M représente un métal alcalin et R a les significations indiquées ci-dessus,
b) avec un agent réducteur et, lorsque X représente -NH-,
c) avec un réactif introduisant le groupe amino.

6. Procédé selon la revendication 5, caractérisé en ce qu'on fait réagir avec un composé de formule Il dans laquelle M représente le caesium.

7. Procédé selon la revendication 5 ou 6, caractérisé en ce que, au stade opératoire b), on réduit à l'aide d'un hydrure complexe ou du diborane.

8. Procédé selon l'une des revendications 5 à 7, caractérisé en ce que l'on fait réagir avec un composé de formule Il dans laquelle R représente le groupe méthyle.

9. Procédé de préparation de peptides et d'amides peptidiques, caractérisé en ce que l'on utilise la résine synthétique définie dans la revendication 1 en tant que support pour la synthèse en phase solide.

10. Procédé selon la revendication 9, caractérisé en ce que l'on prépare des peptides ou des amides peptidiques protégés sur l'azote terminal et/ou sur d'autres groupes fonctionnels.

11. Procédé selon la revendication 9 ou 10, caractérisé en ce que :
a) on fait réagir la résine synthétique avec un composé de formule W¹-AM¹-Y-H dans laquelle Y représente -O- ou -NH-, W¹ représente un groupe protecteur du groupe amino de l'azote terminal et AM¹ représente le radical acyle d'une séquence d'aminoacides éventuellement protégée sur les groupes fonctionnels et consistant en 1 à 25 radicaux d'aminoacides, ou avec un dérivé fonctionnel réactif de cette séquence en vue d'ancrer le radical W¹-AM¹ sur le groupe -X- de la résine,
b) on scinde le groupe protecteur de l'azote terminal W¹,
c) on acyle le groupe amino libéré de l'azote terminal par réaction avec un acide de formule W²-AM²-OH dans laquelle W² et AM² ont des significations analogues à celles des symboles W¹ et AM¹ définis ci-dessus, ou avec un dérivé réactif d'un tel acide,
d) on répète les opérations successives de scission selon b) et d'acylation selon c) à volonté jusqu'à obtention de la séquence d'aminoacides recherchée,
e) on sépare le peptide ou l'amide peptidique formé de la résine par acidolyse, et si on le désire après scission des groupes protecteurs ou avec scission simultanée des groupes protecteurs.

12. Procédé selon la revendication 11, caractérisé en ce que, pour la protection du groupe amino de l'azote terminal, on utilise un groupe protecteur du groupe amino W¹ ou W² respectivement scindable en milieu basique et on procède à la scission du stade opératoire b) à l'aide d'une base.

13. Procédé selon la revendication 12, caractérisé en ce que l'on utilise en tant que groupe protecteur du groupe amino scindable en milieu basique W¹ ou W² le groupe 9-fluorénylméthoxycarbonyle (Fmoc).

14. Procédé selon l'une des revendications 11 à 13, caractérisé en ce que, au stade opératoire a), on fait réagir une résine synthétique pour laquelle X représente -O- avec un acide de formule W¹-AM¹-OH dans laquelle W¹ et AM¹ ont les significations indiquées dans la revendication 11, ou avec un dérivé fonctionnel réactif d'un tel acide, et on sépare le produit final sous la forme acide.

15. Procédé selon l'une des revendications 11 à 13, caractérisé en ce que, au stade opératoire a), on fait réagir une résine synthétique pour laquelle X représente -O- avec un amide de formule W¹-AM¹-NH₂ dans laquelle W¹ et AM¹ ont les significations indiquées dans la revendication 11, et on sépare le produit final à l'état d'amide peptidique.

16. Procédé selon l'une des revendicatrions 11 à 13, caractérisé en ce que, au stade opératoire a), on fait réagir une résine synthétique pour laquelle X représente -NH- avec un acide de formule W¹-AM¹-OH dans laquelle W¹ et AM¹ ont les significations indiquées dans la revendication 11, ou avec un dérivé fonctionnel d'un tel acide, et on sépare le produit final à l'état d'amide peptidique.

17. Utilisation d'une résine synthétique selon revendication 1 en tant que support pour la synthèse en phase solide de peptides et d'amides peptidiques.

18. Une résine synthétique ayant la structure définie dans la revendication 1, et qui porte, à la place du groupe -X-H, un groupe -NH-W, -X-AM-W ou -X-AM-H dans lequel -X- représente -O- ou -NH-, W représente un groupe protecteur du groupe amino de l'azote terminal et AM représente le radical acyle d'une séquence d'aminoacides protégée si on le désire sur les groupes fonctionnels et consistant en 1 à 180 radicaux d'aminoacides.

19. Un peptide ou un amide peptidique obtenu par le procédé selon revendication 11, de formule W-AM⁰-XH ou H-AM⁰-XH dans laquelle -X- représente -O- ou -NH-, W représente un groupe protecteur du groupe amino de l'azote terminal et AM⁰ le radical acyle d'une séquence d'aminoacides protégée si on le désire sur les groupes fonctionnels et consistant en 2 à 180 radicaux d'aminoacides.

## Revendications (Revendications pour l'(les) Etat(s) contractant(s) suivant(s): ES)

1. Procédé de préparation d'une résine synthétique à base d'un polystyrène réticulé par 0 à 5 mol% de divinylbenzène et utilisable en tant que support pour des synthèses de peptides en phase solide, qui est substituée sur les noyaux benzéniques de son squelette par les groupes de formule 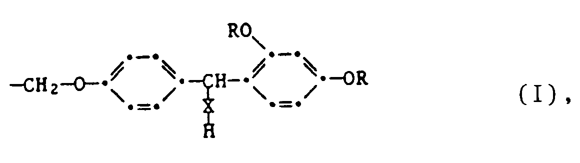 dans laquelle X représente -O- ou -NH- et R représente un groupe alkyle en C 1-C 4, caractérisé en ce que, partant d'un polystyrène réticulé par 0 à 5% de divinylbenzène et chlorométhylé ou bromométhylé sur les noyaux benzéniques du squelette, lui-même utilisable en tant que support pour la synthèse des peptides en phase solide, on le fait réagir successivement
a) avec un composé de formule 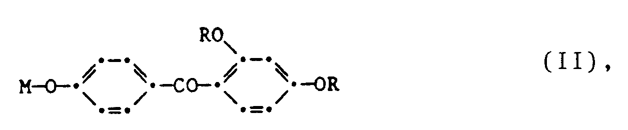 dans laquelle M représente un métal alcalin et R a les significations indiquées ci-dessus,
b) avec un agent réducteur et, lorsque X représente -NH-,
c) avec un réactif introduisant le groupe amino.

2. Procédé selon la revendication 1, caractérisé en ce que l'on utilise un polystyrène réticulé par 1 à 2% de divinylbenzène.

3. Procédé selon la revendication 1 ou 2, caractérisé en ce que l'on fait réagir avec un composé de formule II dans laquelle M représente le caesium.

4. Procédé selon l'une des revendications 1 à 3, caractérisé en ce que, au stade opératoire b), on réduit à l'aide d'un hydrure complexe ou du diborane.

5. Procédé selon l'une des revendications 1 à 4, caractérisé en ce que l'on fait réagir avec un composé de formule II dans laquelle R représente le groupe méthyle.

6. Procédé selon la revendication 5, pour la préparation d'une résine synthétique pour laquelle, dans la formule I, X représente -O- et R un groupe méthyle.

7. Procédé selon la revendication 5, pour la préparation d'une résine synthétique pour laquelle, dans la formule I, X représente -NH- et R un groupe méthyle.

8. Procédé de préparation de peptides et d'amides peptidiques, caractérisé en ce que l'on utilise une résine synthétique définie dans la revendication 1 en tant que support pour la synthèse en phase solide.

9. Procédé selon la revendication 8, caractérisé en ce que l'on prépare des peptides ou amides peptidiques protégés sur l'azote terminal et/ou sur d'autres groupes fonctionnels.

10. Procédé selon la revendication 8 ou 9, caractérisé en ce que :
a) on fait réagir la résine synthétique avec un composé de formule W¹-AM¹-Y-H dans laquelle Y représente -O- ou -NH-, W¹ représente un groupe protecteur du groupe amino de l'azote terminal et AM¹ représente le radical acyle d'une séquence d'aminoacides éventuellement protégée sur les groupes fonctionnels et consistant en 1 à 25 radicaux d'aminoacides, ou avec un dérivé fonctionnel réactif de cette séquence en vue d'ancrer le radical W¹-AM¹ sur le groupe -X- de la résine,
b) on scinde le groupe protecteur de l'azote terminal W¹,
c) on acyle le groupe amino libéré de l'azote terminal par réaction avec un acide de formule W²-AM²-OH dans laquelle W² et AM² ont des significations analogues à celles des symboles W¹ et AM¹ définis ci-dessus, ou avec un dérivé réactif d'un tel acide,
d) on répète les opérations successives de scission selon b) et d'acylation selon c) à volonté jusqu'à obtention de la séquence d'aminoacides recherchée,
e) on sépare le peptide ou l'amide peptidique formé de la résine par acidolyse, et si on le désire après scission des groupes protecteurs ou avec scission simultanée des groupes protecteurs.

11. Procédé selon la revendication 10, caractérisé en ce que, pour la protection du groupe amino de l'azote terminal, on utilise un groupe protecteur du groupe amino W¹ ou W² respectivement scindable en milieu basique et on procède à la scission du stade opératoire b) à l'aide d'une base.

12. Procédé selon la revendication 11, caractérisé en ce que l'on utilise en tant que groupe protecteur du groupe amino scindable en milieu basique W¹ ou W² le groupe 9-fluorénylméthoxycarbonyle (Fmoc).

13. Procédé selon l'une des revendications 10 à 12, caractérisé en ce que, au stade opératoire a), on fait réagir une résine synthétique pour laquelle X représente -O- avec un acide de formule W¹-AM¹-OH dans laquelle W¹ et AM¹ ont les significations indiquées dans la revendication 11, ou avec un dérivé fonctionnel réactif d'un tel acide, et on sépare le produit final sous la forme acide.

14. Procédé selon l'une des revendications 10 à 12, caractérisé en ce que, au stade opératoire a), on fait réagir une résine synthétique pour laquelle X représente -O- avec un ami de de formule W¹-AM¹-NH₂ dans laquelle W¹ et AM¹ ont les significations indiquées dans la revendication 11, et on sépare le produit final à l'état d'amide peptidique.

15. Procédé selon l'une des revendications 10 à 12, caractérisé en ce que, au stade opératoire a), on fait réagir une résine synthétique pour laquelle X représente -NH- avec un acide de formule W¹-AM¹-OH dans laquelle W¹ et AM¹ ont les significations indiquées dans la revendication 11, ou avec un dérivé fonctionnel d'un tel acide, et on sépare le produit final à l'état d'amide peptidique.

16. Procédé selon revendication 8 ou 9, caractérisé en ce que les peptides ou amides peptidiques consistent en 2 à 180 radicaux d'aminoacides.

17. Procédé de préparation d'une résine synthétique de structure définie dans la revendication 1, mais qui porte à la place du groupe -X-H, un groupe -X-AM-W ou -X-AM-H dans lequel -X- représente -O- ou -NH-, W représente un groupe protecteur du groupe amino de l'azote terminal et AM le radical acyle d'une séquence d'aminoacides protégée si on le désire sur les groupes fonctionnels et qui consiste en 1 à 180 radicaux d'aminoacides, caractérisé en ce que l'on fait réagir une résine synthétique telle que définie dans la revendication 1
a) avec un composé de formule W¹-AM¹-Y-H dans laquelle Y représente -O- ou -NH-, W¹ représente un groupe protecteur du groupe amino de l'azote terminal et AM¹ le radical acyle d'une séquence d'aminoacides protégée si on le désire sur les groupes fonctionnels et consistant en 1 à 25 radicaux d'aminoacides, ou avec un dérivé fonctionnel réactif d'une telle séquence, en vue d'ancrer le radical W¹-AM¹-sur le groupe -X- de la résine, le cas échéant
b) on scinde le groupe protecteur W¹ de l'azote terminal, le cas échéant
c) on acyle le groupe amino libéré sur l'azote terminal par réaction avec un acide de formule W²-AM²-OH dans laquelle W² et AM² ont des significations analogues à celles des symmboles W¹ et AM¹ définis ci-dessus, ou avec un dérivé réactif fonctionnel d'un tel acide, et
d) on répète les opérations alternatives de scission selon b) et d'acylation selon c) à volonté jusqu'à obtention de la séquence d'aminoacides voulue.

18. Procédé de préparation d'une résine synthétique de structure définie dans la revendication 1, mais qui, à la place du groupe -X-H, porte le groupe -NH-W dans lequel W représente un groupe protecteur du groupe amino sur l'azote terminal, caractérisé en ce que l'on fait réagir une résine synthétique définie dans la revendication 1 avec un composé de formule W-NH₂ dans laquelle W a les significations indiquées ci-dessus, ou avec un dérivé fonctionnel réactif d'un tel composé, en vue d'ancrer le radical W-NH- sur le groupe -X- de la résine.
